(19) Europäisches Patentamt

European Patent Office

Office européen des brevets

(11) Veröffentlichungsnummer: **0 215 731 B1**

(12) **EUROPÄISCHE PATENTSCHRIFT**

(45) Veröffentlichungstag der Patentschrift: **30.12.92**

(21) Anmeldenummer: **86730142.6**

(22) Anmeldetag: **17.09.86**

(51) Int. Cl.⁵: **A61N 1/365**

(54) **Herzschrittmacher.**

(30) Priorität: **17.09.85 DE 3533501**

(43) Veröffentlichungstag der Anmeldung:
**25.03.87 Patentblatt 87/13**

(45) Bekanntmachung des Hinweises auf die
Patenterteilung:
**30.12.92 Patentblatt 92/53**

(84) Benannte Vertragsstaaten:
**DE FR GB IT NL SE**

(56) Entgegenhaltungen:
**EP-A- 0 064 940     EP-A- 0 080 347
EP-A- 0 089 014     EP-A- 0 108 360
EP-A- 0 140 472     DE-A- 2 058 009
DE-A- 2 520 730**

(73) Patentinhaber: **BIOTRONIK Mess- und Therapiegeräte GmbH & Co Ingenieurbüro Berlin
Sieversufer 8
W-1000 Berlin 47(DE)**

(72) Erfinder: **Schaldach, Max
Turnstrasse 5
W-8520 Erlangen(DE)**

(74) Vertreter: **Christiansen, Henning, Dipl.-Ing.
Patentanwalt CHRISTIANSEN Pacelliallee
43/45
W-1000 Berlin 33(DE)**

**Beschreibung**

Die Erfindung betrifft einen Herzschrittmacher der im Oberbegriff des Anspruchs 1 angegebenen Art.

Bekannte Herzschrittmacher werden einmalig fest oder durch Programmierung auf ihre Betriebsbedingungen eingestellt.

Dabei ist jedoch nicht möglich, die Meßwertaufnehmer oder die internen Meßwertverarbeitungswege den besonderen und gegebenenfalls wechselnden Betriebsbedingungen anzupassen.

Der im kennzeichnenden Teil des Anspruchs 1 angegebenen Erfindung liegt die Aufgabe Zugrunde, bei einen Herzschrittmacher der eingangs genannten Gattung derart auszubilden, daß Meßwertaufnehmer und die nachfolgenden Verarbeitungswege an die individuellen Gegebenheiten angepaßt und insbesondere auch bei Änderungen der Betriebsbedingungen anpaßbar sind.

Besonders vorteilhaft ist es dabei, wenn externe Meßwege zur Eichung verwendet werden, welche es gestatten mit anderen Meßmethoden oder genaueren Geräten sicherzustellen, daß die schrittmacherinternen Meßmittel exakt eingestellt werden. Insbesondere ist es in günstiger Weise möglich, extern direkt die körperliche Belastung zu erfassen, welche durch die Meßmittel des Schrittmachers nur indirekt ermittelt werden kann. Auf diese Weise wird der menschliche Körper als in den Meßweg einbezogen "eingeeicht". Dabei ist es weiterhin vorteilhaft, wenn dabei auch zeitabhängige Einflußgrößen durch Einbeziehung von zeitabhängig veränderlichen Komponenten oder Systemteilen mit kompensiert werden.

Es ist also eine äußere Einflußgröße bekannt, welche Stimulationsparameter beeinflußt. Diese äußere Einflußgröße ist aber im normalen Betriebsfall nicht erfaßbar, sie kann aber mittelbar während des Stimulationsbetriebs aus anderen physiologischen Meßgrößen ermittelt werden.

Bei der äußeren Einflußgröße handelt es sich vorzugsweise um die körperliche Belastung, die vom Schrittmacher nicht direkt erfaßt werden kann. In einer "Eichphase" werden also die Belastungsdaten dem Schrittmacher mittels externer Programmierungsmittel zugeführt und in Zuordnung zu den physiologischen Meßgrößen abgespeichert. Das geschieht bevorzugt in tabellarischer Form, so daß nach Fortfall der äusseren Einflußgrößen nach Ablauf der Eichphase die durch die physiologischen Größen ermittelten Werte an die Stelle der äußeren Einflußgrößen treten und die Steuerung des Schrittmachers übernehmen. Dazu gehört insbesondere die belastungsabhängige Regelung der Herzfrequenz. Soll das Herzzeitvolumen der äußeren Belastung angepaßt werden, das heißt (gegebenenfalls unter Einfluß einer Zeitkonstante) Proportionalität erreicht werden, so wird mittels einer ersten Tabelle (oder der Programmierung des entsprechenden funktionalen Zusammenhangs in einem digitalen Prozessor) der Zusammenhang von Belastung und Herzzeitvolumen festgelegt. Im Falle der tabellarischen Zuordnung wird in jedem Speicherplatz, unter Umformung des Belastungsmeßwerts als Adresse, ein Wert für das Zeitvolumen abgespeichert, das in Abhängigkeit von der anstehenden Belastung in der Eichphase eingestellt wird. Dabei wird bei konstant angenommenen Schlagvolumen in einer einfachsten Ausführung die Stimulationsfrequenz linear mit der Belastung verändert.

In der Eichphase wird gleichzeitig eine Konversionstabelle in der Form generiert, daß in einem zusätzlichen Speicherplatz, der dem mittels des Belastungswerts adressierten Speicherplatz zugeordnet ist, derjenige physiologische Meßwert abgespeichert wird, welcher zu der betreffenden Belastung gehört. Nach Ablauf der Eichphase wird das Betriebsverhalten des Schrittmachers dann in der Weise umgeschaltet, daß für jede physiologische Meßgröße durch einen Such-(und gegebenenfalls Interpolations)vorgang derjenige Speicherplatz für die Herzzeitvolumengröße aufgesucht wird, welcher diesen (oder einen möglichst nahekommenden) physiologischen Meßwert als gespeicherte digitale Größe enthält. Der in dem durch dieselbe Belastungsgröße adressierbaren zweiten Speicherplatz festgehaltene Herzzeitvolumenwert wird dann dem Schrittmacher vorgegeben und in dem angenommenen Beispiel die zugeordnete Frequenz eingestellt.

Bei einer anderen Variante wird eine neue tabellarische Verknüpfung durch die Adressierung entsprechender Speicherplätze in der Weise geschaffen, daß ein der physiologischen Meßgröße über die Belastung zugeordneten Speicherwert in einem solchen Speicherplatz abgelegt wird, der durch den jeweiligen Zahlenwert der Meßgröße adressiert wird.

Eine Variation der zuvor beschriebenen Lösung liegt darin, daß die physiologischen Meßgrößen direkt der Steuergröße im Schrittmacher zugeordnet wird, ohne das zunächst ein Parameter eingeführt wird, der anschließend durch Umschaltung von der Meßphase in die Betriebsphase eliminiert wird.

Durch die Benutzung einer Zwischentabelle kann jedoch eine zusätzlich Kompensation der vorgefundenen Nichtlinearitäten der Meßwertaufnehmer erfolgen und das entsprechende Signal auch unabhängig zur Adressierung weiterer Kennfeldspeicher benutzt werden.

Bei einer weiteren Ausführung braucht der Zusammenhang zwischen physiologischen Meßwerten und gewünschtem Herzverhalten nicht über eine äußere Einflußgröße als Parameter festgelegt zu werden. Treten nämlich Spontanaktivitäten über genügend lange Zeiträume auf, so daß eine Aussage über einen genügend großen Belastungsbereich erfolgen kann, so läßt sich die Zuordnung physiologische Meßgröße (als Maß für die Belastung) und Stimulationsverhalten (vorzugsweise Herzrate) direkt auf die genannte Weise vornehmen. Dabei dient eine im Spontanbetrieb aufgenommene Tabelle in Form der Zuordnung des das Herzzeitvolumen kennzeichnenden Inhaltes eines Speicherplatzes (vorzugsweise die Frequenz) zu einem physiologischen Meßwert, dessen Zahlenwert diesen Speicherplatz adressiert im Stimulationsbetrieb als Bezugsgröße für das bei vorliegenden betreffenden physiologischen Meßgrößen der erreichende Herzzeitvolumen (wobei insbesondere also die Herzfrequenz so gesteuert wird, wie es dem natürlichen physiologischen Verhalten in der Eichphase (hier Spontanaktivität) entspricht.

Bei anderen Weiterbildungen der Erfindung werden die mittels Meßwertaufnehmern enthaltenden, die jeweiligen physiologischen Parameter kennzeichnenden Meßgrößen einer Analog/Digital-Wandlung unterzogen und der erhaltene Digitalwert bildet - mindestens mittelbar - die Adresse oder eine Adresseninformation zur Auswahl der gespeicherten Ausgangsdaten, die in Speicherplätzen eines veränderlichen Speichers festgehalten werden können, so daß der funktionale Zusammenhang für einen Satz Ausgangsgrößen durch Veränderung der Speicherinhalte frei festgelegt werden kann.

Durch die Maßnahmen nach der Erfindung ergibt sich die vorteilhafte Möglichkeit, die Eingangsgrößen mehrerer Meßwertaufnehmer für dieselbe physiologische Größe miteinander zu verknüpfen. Diese besteht darin, daß die Teiladressen zur Adressierung der Speicherplätze des einen Speichers durch Zusammensetzung der aus den Meßwerten gebildeten Digitalwerte zu einer gemeinsamen Digitalwert erzeugt werden.

Es soll hinzugefügt werden, daß die Tabellierung des funktionalen Zusammenhangs selbstverständlich nicht ausschließlich direkt durch Zuordnung von Wertepaaren, d.h. durch Abspeicherung eines dem jeweiligen Absolutwert entsprechenden Größe in dem jeweiligen Speicherplatz vorgenommen werden muß, sondern daß die Speicherplätze anstelle des Absolutwerts auch andere Information über den funktionalen Zusammenhang - beispielsweise als Steigungsmaß oder als jeweilige Differenzen zu einem Bezugswert - enthalten kann.

Eine andere Möglichkeit der Verknüpfung zweier denselben physiologischen Parameter betreffenden Größen besteht darin, daß die digitalisierten Werte jeweils einer mathematischen Mittelung unterzogen werden und die gemittelten Ausgangswerte der Weiterverarbeitung unterzogen werden.

Durch die zuvor beschriebenen Maßnahmen der tabellierten Verarbeitung von nichtlinearen funktionalen Zusammenhängen und deren Verknüpfung ist es möglich, die vom Körper abgeleiteten physiologischen Meßgrößen in einer Art und Weise zu verarbeiten, welche dem behandelnden Arzt stets eine gute Übersicht über die aktuellen Signalzustände gibt.

Bei einer anderen bevorzugten Ausführung ist neben den in den programmierbaren Kennfeldspeichern noch jeweils ein entsprechender Festwertspeicher vorgesehen, der Erfahrungswerte enthält, auf die bei einer großen Zahl von Patienten zurückgegriffen werden kann, so daß, wenn die übrigen veränderlichen Speicher noch nicht programmiert sind, trotzdem ein eingeschränkter Betrieb möglich ist. Das Umschalten zum Auslesen von Speicherwerten aus diesen jeweils zugeordneten Festwertspeichern kann durch Änderung eines einzigen Bits (Speicherauswahl-Bit) erfolgen, so daß auch in einem eventuellen Störfall mindestens ein Notbetrieb möglich ist.

Ein weiteres Problem bei der Verarbeitung einer im Körper ableitbaren Größe besteht darin, daß bei dieser Signalverknüpfung vielfach auch der zeitabhängige Verlauf von Eingangs- und Ausgangsgrößen berücksichtigt werden muß. Da die im Vorstehenden dargestellte tabellarische Verknüpfung im Kennfeld zwar die Berücksichtigung nichtlinearer Zusammenhänge zuläßt, können jedoch zeitliche Vor- bzw. Nachwirkungen nicht ohne weiteres berücksichtigt werden, auch würde die Programmierung nichtlinearer zeitabhängiger Vorgänge die Übersichtlichkeit sehr stark beeinträchtigen bzw. wäre aufgrund der komplexen zu berücksichtigenden zusammenhänge mit zumutbarem Aufwand überhaupt nicht ausführbar.

Gemäß der bevorzugten Ausführung wird nun gegebenenfalls jeweils im digitalen Verarbeitungszweig vor bzw. nach einem tabelliert adressierbaren Speicher ein lineares Zeitglied eingeschaltet, welches eine oder mehrere Zeitkonstanten simuliert. Dabei ist die Art der Zeitkonstante(n) bzw. das entsprechende Verhalten eines Regelgliedes (D- oder I-Verhalten) oder ein Verzögerungszeit durch Eingabe der betreffenden Parameter programmierbar. Neben der physikalischen Realisierung durch analoge Bauelemente (aktive Übertragungsglieder mit Speichergliedern und Widerständen, wobei die Widerstände durch die Programmierung veränderbar sind, wird die digitale Nachbildung durch einen entsprechenden Rechner, der die zugehörige Übertragungsfunktion digital auswertet bevorzugt. Bei dieser Weiterbildung der Erfindung wird in günstiger Weise die Erkenntnis berücksichtigt, daß bei einer Anordnung, welche aufgrund von zusätzlich im Körper ableitbaren Größen ein Ausgangssignal zur elektrischen Stimulation des Herzens bildet, eine genügende Genauigkeit auch dann erzielt werden kann, wenn die zeitabhängigen und die nichtlinearen Teile der Übertragungsfunktionen konzentriert und getrennt verarbeitet werden.

Derartige Zeitkonstanten tragen der Tatsache Rechnung, daß bei einigen physiologischen im Körper ableitbaren Meßgrößen Mengen von Körperflüssigkeiten- oder anderen Substanzmengen beteiligt sind, bei denen es eine gewisse Zeit erfordert, bis sie gemeinsam gleichmäßig die physikalische oder chemische Meßgröße annehmen. Als Beispiel dafür sei die Bluttemperatur oder die Blutsauerstoffsättigung, der pH-Wert des Blutes, die systolischen Intervalle etc. genannt.

Durch die Trennung der Einstellung von Zeitkonstanten und der übrigen tabellarischen Programmierung lassen sich beim Abgleich der die Signalverarbeitung kennzeichnenden Werte durch entsprechende Programmierung oder bei Selbstabgleich des Systems gezielt diejenigen Werte verändern, welche das betreffende System wesentlich kennzeichnen.

So ist es bei der Auswertung der Körpertemperatur zur Auswertung in Richtung auf eine die Belastung des Patienten kennzeichnenden Größe vorteilhaft, durch Verarbeitung des Meßwertes für die Körpertemperatur (in digitaler Form) zunächst eine differenzierende Komponente anzubringen, um die integrierende am Wärmetransport beteiligten Blutmenge zu kompensieren.

Um die Störsicherheit der Anordnung zu vergrößern, sind Maßnahmen vorgesehen, welche Fehler erkennen und in ihrer Auswirkung dadurch eliminieren, daß auf Ersatzfunktionen umgeschaltet wird.

Zwei Meßwertaufnehmer für die Bluttemperatur, von denen einer in einem peripheren Teil des Kreislaufs angeordnet und der andere im Bereich des Körperzentrums vorgesehen ist, liefern unterschiedliche Meßwerte, wenn sich der körperliche Belastungszustand ändert. Im Zustand der Ruhe liefern beide Aufnehmer jedoch nach einiger Zeit gleiche Werte, wenn sie intakt sind. Eine Kontrolle läßt sich also auch bei einem implantierten System durchführen, wenn der Patient dazu jeweils in Ruhe (und gegebenenfalls bei unterschiedlichen Belastungen) untersucht wird. Die Kontrolle des zeitlich stationären Verhaltens eines Meßwertes und damit eines stationären Ausgagssignals des Meßwertgebers ermöglicht eine Baugruppe zur zeitlichen Mittelwertbildung, welche nur diejenigen Signale als gültig ausgibt, die begrenzten zeitlichen Schwankungen unterliegen. Somit sind also die Signale der beiden vorgenannten Meßwertaufnehmer für die Bluttemperatur im stationären Fall redundant, und lassen Rückschlüsse auf ihre Funktion zu bzw. eine gegenseitige Eichung.

Zur Eichung stehen weiterhin neben einem ergometrisch aufgenommenen Signal als zusätzliches Bezugssignal die Herzeigenaktionen bei nicht intervenierendem Betrieb zur Verfügung.

Eine entsprechende Kontrolle der von einem Meßwertaufnehmer gelieferten Signale ergibt sich aus der Berücksichtigung der Tatsache, daß die sich im Körper einstellenden, physiologische Größen kennzeichnenden Signale nur mit einer begrenzten zeitlichen Rate ändern können. Weichen also Signalwerte in starkem Maße von unmittelbar vorangegangenen Signalen stark ab, so ist damit ebenfalls ein Indiz für eine Fehlfunktion gegeben.

Im Voranstehenden wurde bereits erwähnt, daß verschiedene Meßwertaufnehmer für dieselbe physiologische Größe (beispielsweise die Körpertemperatur) an verschiedenen Stellen des Körpers vorgesehen sein können, um eine erhöhte Sicherheit bei der Erfassung zu ermöglichen. Hierbei werden aber auch zusätzliche Informationen geliefert, welche die zeitliche Steuerung beeinflussen können. Dabei ist zunächst einmal vorauszuschicken, daß gemäß einer bevorzugten Ausführung der Erfindung die Signale derartiger unterschiedlich im Körperkreislauf plazierten Meßwertaufnehmer bei der Verarbeitung durch das hier dargestellte System vor der Zusammenführung in einer den physiologischen Verhältnissen entsprechenden Weise aneinander angepaßt werden. Dabei ist durch eine geeignete Zeitkonstante und durch einen abspeicherbaren - gegebenenfalls nichtlinearen - Zusammenhang ein Teil des physikalischen körpereigenen Regelsystems nachbildbar.

Die Eichung läßt sich mit dem hier dargestellten System bevorzugt in der Weise vornehmen, daß zunächst bei stationärer Belastung eine Stimulationsrate eingestellt wird, welche dem betreffenden Belastungsfall entspricht, wobei auch die natürliche Herztätigkeit des Patienten zu früheren Zeitpunkten als Vergleich herangezogen werden kann.

Bei anderen vorteilhaften Weiterbildungen der Erfindung ist das Vorsehen weiterer - gegebenenfalls externer - Meßwertaufnehmer, die mittels der interaktiven Kommunikation der Programmiermittel mit dem hier dargestellten System zum Informationsaustausch verbunden sind - günstig, wobei im Rahmen der vollständigen Programmierbarkeit des Systems auch die Verknüpfung der Signalflußwege bzw. der Einflußgrößen vorwählbar ist. Auf diese Weise kann das Verhalten eines bereits implantierten Schrittmachers sowohl den weiteren Erkenntnissen der behandelnden Ärzte über den Patienten allgemein bzw. dessen weiterer Krankheitsbild angepaßt werden, daneben können aber auch Fortschritte bei der medizinischen Anwendung der hier dargestellten Technik überhaupt nachträglich noch Berücksichtigung finden. Die technische Realisierung der Programmierbarkeit der Verknüpfungen erfolgt bevorzugt in der Weise, daß ein zusätzlicher Speicher nach Art einer Matrix vorgesehen ist, in dem für eine Anzahl der vorstehend beschriebenen Baugruppen (Tabellenspeicher, Zeitkonstantenspeicher, Verknüpfungsbausteine, Mittelwertbildner) Speicherplätze vorgesehen sind, welche die Adressen von Speicherplätzen aufnehmen, in denen die Ein- bzw. Ausgangsdaten für diese Bausteine abzulegen sind.

Die die Elektrostimulation kennzeichnenden Parameter wirken sich mit unterschiedliche Intensität auf das Stimulationsverhalten des Schrittmachers aus, wobei die Stimulation bervorzugt in der Weise beeinflußt wird, daß die Herzleistung der aktuellen körperlichen Belastung angepaßt wird.

Eine dem letzten Verknüpfungselement nachgeschaltete Baugruppe dient dazu, die Stimulation des Herzens beeinflussende Größe derart anzupassen, daß die aufgrund der Stimulation veränderte Leistung des Herzens (vorzugsweise über die Stimulationsrate) dem aufgrund der physiologisch im Körper ermittelten Paramter nur innerhalb desjenigen Leistungsbereichs angepaßt wird, den das Herz des Patienten zu überstreichen in der Lage ist.

Es ist ersichtlich, wie sich mittels des hier dargestellten Systems für den Arzt in übersichtlicher Form das gesamte Reaktions- und Steuerverhalten in anschaulicher Weise programmieren und überblicken läßt.

Die Anpassung an bestimmte Therapieformen ist ohne Schwierigkeiten möglich, wobei beispielsweise beim ischämischen Herzen diejenigen Frequenzbereiche, in denen das Herz eine adäquate Leistungsfähigkeit zeigt, durch Stimulation mit der betreffenden Rate bei nichtspontaner Herzaktivität durch Programmierung gezielt "anwählbar" sind, so daß im Betrieb aufgrund der nichtlinearen Eigenschaften des Systems jene "leistungsfähigen Arbeitsbereiche" des Herzens aufgesucht werden.

Vorteilhafte Weiterbildungen des Ausführungsbeispiels sind in den Unteransprüchen gekennzeichnet bzw. werden bei der nachstehenden Darstellung zusammen mit einer bevorzugten Ausführung der Erfindung näher beschrieben. Es zeigen:

Fig. 1 die Systemstruktur eines Ausführungsbeispiels der Erfindung,

Fig. 2 ein im Speicherbereich des Ausführungsbeipiels benutztes Kennfeld,

Fig. 3 die in einem Signalverarbeitungsbaustein vorhandenen Elemente unter Einschluß eines Kennfeldes nach Figur 2,

Fig. 4 eine Anordnung von Signalverarbeitungsbausteinen zur programmierbaren Verknüpfung,

Fig. 4a eine Hilfsbaustein zur Signalspeicherung und Verknüpfung im Zusammenhang mit den Anordnungen gemäß Figuren 3 und 4,

Fig. 5 ein gemäß der Erfindung mit den vorstehenden Bausteinen realisierter Herzschrittmacher in Blockdarstellung

Fig. 5a ein Bild zur Veranschaulichung des Zusammenwirkens der Kommunikationseinheit mit den Bausteinen gemäß Figuren 4 und 4a sowie

Fig. 6a bis 6e verschiedene Kennfelder in räumlicher Darstellung zur Erläuterung verschiedener Varianten des Ausführungsbeispiels.

Bei dem nachstehend beschriebenen Ausführungsbeispiel eines Herzschrittmachers gemäß der Erfindung werden verschiedene Baugruppen, welche der Signalbearbeitung dienen und als matrixartig organisierte bzw. Kennfelder enthaltende Speicher beschrieben sind, in ähnlicher Konfiguration mehrfach benutzt, so daß es für das Verständnis ausreichend ist, wenn diese Grundbaugruppen in ihrer Funktion allgemein beschrieben werden. Bei den späteren Darstellungen des funktionalen Zusammenwirkens der Gesamtanordnung entsprechend den wiedergegebenen Blockschaltbildern kann dann auf die Beschreibung von Einzelheiten der Funktionsweise dieser Baugruppen verzichtet werden.

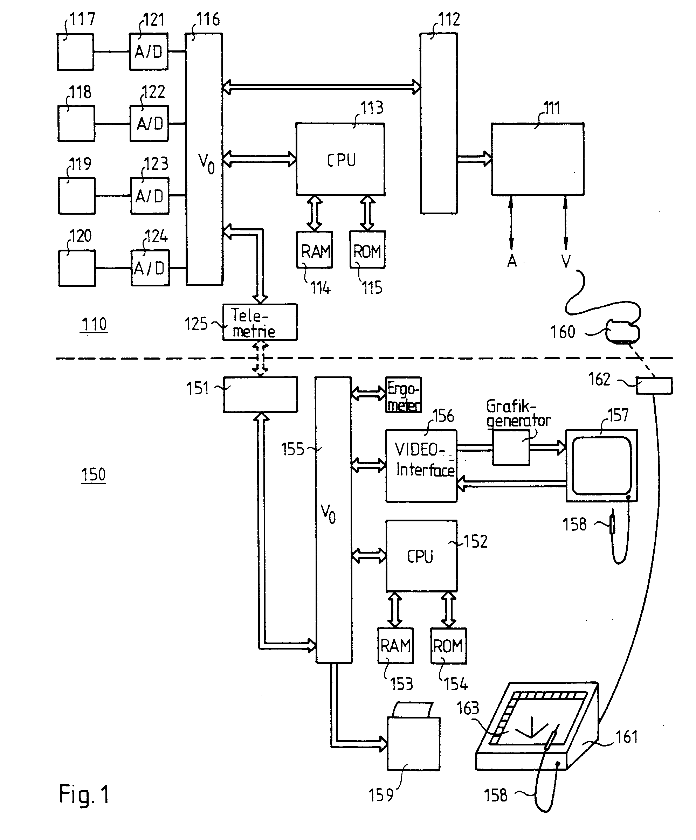

In Figur 1 ist zunächst einmal der grundsätzliche Aufbau eines Herzschrittmachers mit Mitteln zur Verarbeitung minstens einer Eingangs-Meßgröße zur physiologischen Beeinflussung vorzugsweise der Herzrate dargestellt.

Eingangsseitig ist also mindestens ein Meßwertaufnehmer für eine innerhalb oder außerhalb des Körpers aufnehmbare Meßgröße vorgesehen, welche zur physiologischen Steuerung der Stimulationsrate bzw. des Herzschlaggrundintervalls - im Falle eines Demand-Schrittmachers - heranziehbar ist. Dabei handelt es sich um solche Größen, welche von solchen elektrisch im Herzen ableitbaren Signalen verschieden sind, die jeweils nur zur Verhinderung einer Stimulation in Konkurrenz zu Kammereigenaktionen bzw. zur Synchronisation der Stimulationsimpulse dienen und nur eine aktuelle Herzaktion betreffen. Entsprechend sind sämtliche Stimulationsgrößen in Abhängigkeit von den "physiologischen" Eingangsgrößen veränderbar, für die ein Zusammenhang für ein Stimulationsverhalten auffindbar ist das über mehrere Herzschläge gültig ist. Das sind alle für die Stimulation relevanten Größen, die nicht in Beziehung zu nur einer bestimmten Herzaktion stehen, sondern das Stimulationsverhalten im allgemeinen betreffen. Dazu gehören auch die bei Schrittmachern "programmierbaren" Parameter und auch künftig aufzufindende, welche das Stimulationsverhalten - gemessenan der erzielten Leistungsfähigkeit des Herzens - verbessern.

Entsprechende Meßwertaufnehmer sind in Form von temperaturabhängig veränderlichen Widerständen zur Messung der Bluttemperatur, als Meßelektroden zur Ermittlung von impedanzkardiografischen (elektroplethysmographischen) Daten, als fotoelektrischer Meßwertaufnehmer für die Ermittlung der Blutsauerstoffsättigung mittels einer Lichtschranke, als chemische Sonden zur Ermittlung des pH-Wertes oder als Druck bzw. Schallaufnehmer für Meßwerte, die mit der mechanischen Kontraktion in Zusammenhang stehen, grundsätzlich bekannt. Insbesondere die im Herzen selbst aufgenommenen und ein Maß für die Kammerfüllung bildenden Meßgrößen, die damit bereits mit einer für das Herzzeitvolumen - und damit für die Leistungsfähigkeit des Herzens - kennzeichnenden Größe verknüpft sind, lassen sich in vorteilhafter Weise zur Beeinflußung der Stimulationsparameter (insbesondere der Herzrate) heranziehen.

Aber auch aus den am Herzen aufgenommenen elektrischen Spannungspotentialen können zusätzliche physiologische Meßwerte gewonnen werden, wie beispielsweise das Q-T-Intervall, welches ebenfalls zur Herzfrequenzsteuerung herangezogen werden kann.

Diese Größen werden nachfolgend als "physiologische Meß- oder Eingangsgrößen" bezeichnet. Bei einem mehrere Eingangssignale verarbeitenden Schrittmacher ist demnach von einem "multiphysiologischen Schrittmacher" zu sprechen.

Die analogen Ausgangssignale der Meßwertaufnehmer werden jeweils einem nachgeschalteten Analog-/Digital-Wandler zugeführt, der die Eingangssignale in entsprechende mit den beschriebenen digitalen Speichermitteln bzw. Signalprozessoren verarbeitbare Signale umwandelt. Die Verarbeitung der jeweils einen Meßwert bildenden Datenworte erfolgt dabei mittels eines Mikroprozessors und dessen peripheren Bausteinen zur Datenspeicherung und -verarbeitung.

Bei dem in Figur 1 dargestellten Ausführungsbeispiel ist die physikalische (Hardware-)Struktur des nachfolgend dargestellten Schrittmachersystems wiedergegebenen. Es handelt sich dabei um ein Mikroprozessorsystem, welches mittels Telemetrie mit einer äußeren Steuereinheit 150 als Programmiereinheit bidirektional kommunizieren kann.

Die implantierbare Einheit enthält ein konventionelles multiprogrammierbares Herzschrittmachersystem 111, das über eine oder zwei Elektroden mit dem Ventrikel und/oder dem Atrium in Wechselwirkung tritt. Als Interface zu dem übrigen Schrittmachersystem ist ein Parameterspeicher 112 vorgesehen, welcher gleichzeitig als Puffer für den Datenaustausch mit dem konventionellen Schrittmachersystem dient. In dem Parameterspeicher sind zunächst einmal diejenigen Daten enthalten, welche dem multiprogrammierbaren Schrittmacher 111 als Betriebsparameter, d.h. Steuergrößen, vorgegeben sind. Diese Größen bilden die durch externe Programmierung einstellbaren Werte eines Ein- oder Zweikammerschrittmachers, wobei der Betriebsmodus (von V00 bis DDD) ebenfalls programmierbar ist.

Zusätzlich werden in dem Speicher 112 noch solche Betriebsparameter festgehalten, die über weitere Meßwertaufnehmer im Körper des Patienten aufgenommen und aus "physiologischen" Eingangsgrößen hergeleitet wurden. Im Falle der Q-T-Intervalle können sie aber auch über die Stimulationselektroden selbst abgeleitet sein.

Während das Schrittmachersystem 111 selbst also diejenigen Schaltungen enthält, welche eine Konkurrenz von stimulierenden Ereignissen mit Spontanimpulsen vermeiden, dient das diese Block umgebende Mikroprozessorsystem dazu, weitere Steuergrößen, vorzugsweise die Grundrate, festzulegen und das System 111 insoweit zu ergänzen. Das hat den Vorteil, daß für die Benutzung des Systems 111 auf konventionelle Weise keine zusätzlichen Kenntnisse erforderlich sind, die über diejenigen hinausgehen, welche bei der Benutzung bekannter multiprogrammierbarer Schrittmacher notwendig sind.

Der Schrittmacher 111 ist auch ohne "physiologische" aufgenommene Meßgrößen betriebsfähig, wobei die Grundstimulationsrate entsprechend den konventionellen Schrittmachern durch externe Programmierung vorgegeben wird. Je nach technischer Ausführung lassen sich auch die für den Betrieb des Schrittmacherteils 111 notwendigen Signal- und Datenverarbeitungen durch die CPU 113 des Mikroprozessorsystems vornehmen.

Der CPU 113 zugeordnet sind jeweils ein RAM-Speicher 114 und ein ROM-Speicher 115 sowie eine Ein-/Ausgangseinheit 116, durch die der Datenverkehr mit den peripheren Baugruppen 111 und 125 vorgenommen wird. Dabei ist zu erwähnen, daß mit dem dargestellten System die Signalverarbeitung so erfolgt, wie sie weiter unten beschriebert ist, wobei die dort dargestellten Speicher und Datenverknüpfungen durch entsprechende Programmierung, sozusagen "softwaremäßig" erzeugt werden und in RAM- bzw. ROM-Speicherbereichen vorhanden sind. Das System erscheint dem Benutzer wie eine Anordnung mit der beschriebenen physikalischen Struktur, die entsprechend auch hardwaremäßig realisiert werden könnte. Zur Vermeidung der Darstellung des zeitlichen Nacheinanders bei der Bearbeitung von Programmen in der bei der Datenverarbeitung üblichen Strukturierung, die durch die ausschließlich serielle Verarbeitung durch die CPU in einem Mikroprozessorsystem bedingt ist, erfolgt hier die Beschreibung so, als ob die, betreffenden Speicher separat als Hardware erzeugt und mit den den Datenfluß in den Blockdiagrammen repräsentierenden Steuer- und Datenübertragungsleitungen verbunden wären.

An die Ein-/Ausgangseinheit 116 ist sowohl der Parameterspeicher 112 angeschlossen als auch verschiedene Meßwertaufnehmer 117, 118, 119, 120, welche mittels Digital/Analog-Wandlern 121 bis 124 die aufgenommenen Signale der Einheit 116 zuführen. Die Signalaufnehmer wurden eingangs erwähnt und werden im oder am Körper des Patienten angebracht - insbesondere an der Stimulationselektrode oder dem Gehäuse des Schrittmachers selbst.

Die Signalverarbeitung durch die CPU erfolgt seriell, wobei die einzelnen Baugruppen der nachfolgend zu beschreibenden Struktur gegebenenfalls nach entsprechenden Interrupt-Anforderungen in den Datenaustausch einbezogen werden, so daß die Signalverarbeitung - angepaßt an die zeitlichen Anforderungen der Signale - getaktet quasi gleichzeitig vorgenommen wird.

Mittels der Steuereinheit 150 erfolgt eine Datenübertragung von und zu der implantierten Einheit über eine Telemetrie-Baugruppe 151, welche mit der entsprechenden Telemetrie-Baugruppe 125 kommuniziert.

Die Steuereinheit weist eine eigene CPU 152 auf, die mit eigenen RAM-Speichern 153 bzw. ROM-Speichern 154 Daten austauscht. Außerdem ist eine Ein-/Ausgangseinheit 155 vorhanden, an die die Telemetrie-Einheit 151 und ein Videointerface 156 angeschlossen sind. Das Videointerface steht mit einem Monitor 157 in Verbindung, welches entsprechend dem in der externen Einheit 150 vorhandenen Programm die Abfrage von Daten aus dem implantierbaren System und deren Veränderung ermöglicht. Von besonderer Bedeutung ist, daß die gesamte Bedienerführung und die Mittel zur grafischen Darstellung in der externen Einheit vorhanden sind, einschließlich Daten über die generelle Struktur des implantierbaren Systems. Durch grafische Aufbereitung der erhaltenen Daten und der zur Programmierung des internen Systems notwendigen Festlegungen ist eine komfortable Betriebsweise vorgesehen, welche der Bedienungsperson jederzeit Aufschluß über den Betriebszustand des implantierbaren Systems gibt. Die Programmierung erfolgt dabei mittels eines Lichtgriffels 158, mit dem durch Kontaktierung, des Bildschirms Informationen ausgewählt werden können. Entsprechend ist ein druckempfindlicher Bildschirm verwendbar, bei dem die Auswahl von Informationen durch Berühren der Bildschirmoberfläche möglich ist. Damit kann entsprechend den bekannten Techniken der grafischen Bildverarbeitung eine Auswahl von Bildschirmseiten (entsprechend dem "Blättern" in einer Kartei) erfolgen, wobei sowohl bei der Bedienung als auch bei der Beurteilung der sich im Betrieb einstellenden Daten bevorzugt eine Aufbereitung in grafischen Darstellungen in international verständlichen Symbolen vorgenommen wird. Die einzelnen Funktionsbereiche des implantierbaren Systems sind dabei separat anwählbar, wobei von denjenigen Parameterbereichen, die grundsätzliche Funktionsweise des Schrittmachers betreffen, zu subtileren Verknüpfungen eine Suchbaumstruktur führt, welche mit unterschiedlichen Zugangscodes bis jeweils zu einer dem betreffenden Anwender zugeordneten Tiefe erreichbar ist. Mittels eines Druckers 159 kann ein Protokollausdruck zu Dokumentationszwecken erfolgen.

Die äußere Gestaltung der Baugruppen 110 und 150 ist ebenfalls in Figur 1 wiedergegeben und zeigt, daß der implantierbare Teil die Abmessungen eines normalen Schrittmachers 160 hat, während der externe Kommunikationsteil 161 neben einem auf den Körper des Patienten auflegbaren Antennenteil 162 zusätzlich eine grafische Darstellungsfläche 163 aufweist.

Die nachfolgende Beschreibung bezieht sich auf Einzelheiten der Baugruppe 116 und speziell auf die Verknüpfung der "physiologischen" Eingangsgrößen der Meßweraufnehmer 117 bis 120 sowie die Datenverknüpfung und deren Steuerung.

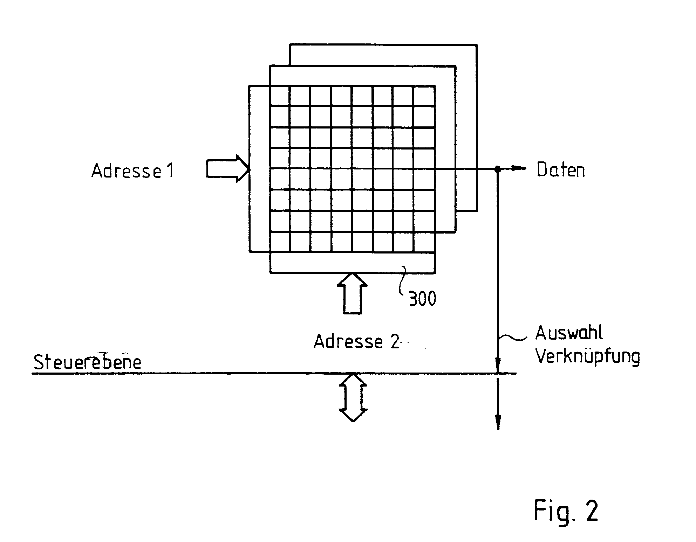

In Figur 2 ist die matrixartige Speicherstruktur eines zur Steuerung des Systems vorgesehenen Kennfelds wiedergegeben, wobei die Speicherplätze der Matrix durch Spaltenund Zeilenadressensignale aufsuchbar sind und der an dem adressierten Speicherplatz vorhandene Zahlenwert ein- bzw. auslesbar ist. Jedes Kennfeld ist mittels eines gemeinsamen Adressenmerkmals aufsuchbar, so daß einerseits eine vereinfachte Adressierung im Normalbetrieb möglich ist, andererseits aber auch eine Neukonfiguration der Verarbeitung und ein ein geschlossenes Auslesen oder Ändern des Inhalts eines Kennfeldes im Zusammenhang mit der externen Kommunikationseinheit ohne weiteres erfolgen kann.

Während im Normalbetrieb eine Verknüpfung der Adresseingänge und Datenein- bzw. Ausgänge nach einem vorgebenen Verknüpfungsschema erfolgt, wobei in vorangehenden Kennfeldspeichern durch Adressierung ausgelesene Daten zur Adressierung nachfolgender Kennfeldspeicher dienen, können diese Daten auch Informationen beihalten, welche die Verknüpfung der Daten aufeinanderfolgender Kennfelder selbst betreffen, sei es bezüglich der Richtung der Verknüpfung oder hinsichtlich bei der Verknüpfung auszuführender (logischer oder mathematischer) Operationen. Die entsprechenden Dateninhalte unterscheiden sich durch zusätzliche Attribute voneinander, welche in entsprechenden nachgeschalteten (nicht dargestellten) Datendiskriminatoren unterschieden werden. Bei der externen Kommunikation ist durch das gemeinsame Adressenmerkmal eines jeden Kennfeldspeichers ein Direktzugriff auf den Inhalt des jeweiligen gesamten Speicherbereiches zum Ein- und Auslesen oder Verändern von Daten möglich. Die Kennfelddaten für die Datenverarbeitung weisen dabei bevorzugt Zugriffsadressen auf, die denjenigen von Hilfsspeichern entsprechend aufrufbar sind, welche ausschließlich Verknüpfungs-, Auswahl- oder sonstige Hilfsinformationen wie von der Kommunikationseinheit auslesbare Datensätze mit Grafik- oder Kenndaten für den betreffenden Schrittmachertyp enthalten.

Dieser Direktzugriff ist durch die Steuer- und Zugriffsebene 306 im unteren Teil der Zeichnung angedeutet. Ein Datenfeld kann also neben einem Baustein der Signalverarbeitung im Sinne der Verknüpfung von Eingangs- und Ausgangssignalen auch einen Speicher bilden, dessen Informationen zum Bestimmen der Aufeinanderfolge bezüglich der Zusammenschaltung von Ein- und Ausgängen von verschiedenen Kennfeldbausteinen bilden.

Die in Figur 2 wiedergegebene Speichereinheit braucht in dieser Form nicht - wie dargestellt - eine physikalische oder hardwaremäßige Einheit im Speicherbereich des Systems zu bilden. Die betreffende Struktur ist auch rein softwaremäßig erzeugbar.

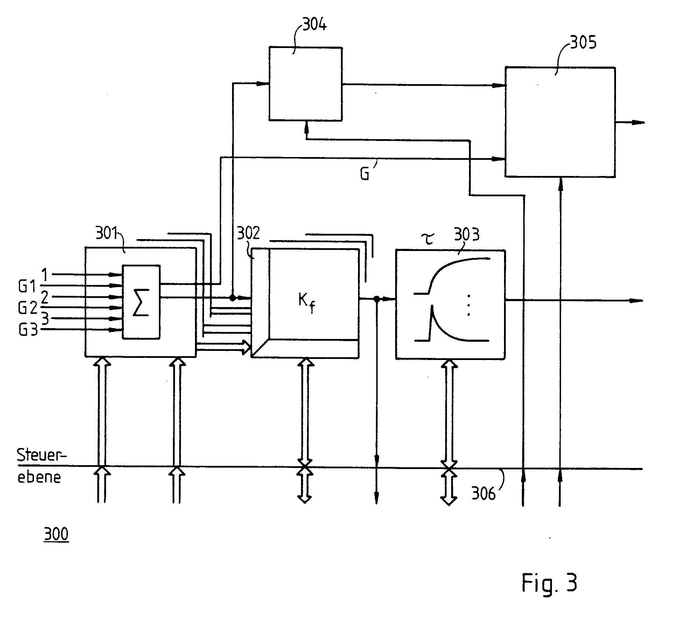

Die Speichereinheit gemäß Figur 2 - die hier synonym auch als "Kennfeldspeicher" oder "Speichermatrix" bezeichnet ist, bildet einen Teil einer in Figur 3 wiedergegebenen "Zelle" als Signalverarbeitungsbaustein, bei der die Speichereinheit noch von Hilfsbaugruppen umgeben ist, welche günstigerweise in einer modulartigen Struktur bei jedem Kennfeld vorgesehen sind und bei der Vernetzung mit verknüpft werden, so daß nur die Ein- und Ausgänge dieser Zelle als Schnittstellen in Erscheinung treten. Bei einer bestimmten Anwendung nicht benötigte Bausteine werden jeweils durch das Laden entsprechender Steuerparamter im Hilfsspeicher (wie weiter unten beschrieben) inaktiv geschaltet.

Die Zusammenfassung der Signale verschiedener Meßwertaufnehmer erfolgt in einem Block 301 unter Berücksichtigung der jedem Signal eines jeden Meßwertaufnehmers zugeordneten Gewichtungssignal. Der Block 301 bildet auch gleichzeitig eine Schalterbaugruppe, welche über die Zugriffsebene 306 verschiedene Signale als Eingangs- (d.h. Adressensignale) auswählt und je nach "Schalterstellung" (beeinflußt über die Auswahlinformation der Steuerebene 306) unterschiedliche Signale mittels des nachfolgenden Kennfeldes zur variablen Adressierung der Ausgabe von Signalen aus dem nachfolgenden Kenfeldspeicher 302 in Form von digitalisierten Amplitudeninformationen verknüpft. Die ausgewählten Eingangssignale bilden jeweils Teiladressensignale, die insgesamt den Speicherbereich des nachfolgenden Kennfeldes überstreichen. Durch dem Block 301 separat zuzuführende Verknüpfungsdaten lassen sich die Eingangsdaten nicht nur schalten, sondern auch (einzeln oder gemeinsam) logischen oder mathematischen Operationen unterziehen, d.h. es können Daten ausgewählt, verschoben oder in sonstiger Weise "bearbeitet" werden. Insbesondere sind auf diese Weise Arbeitsbereiche oder Arbeitskennlinien auswählbar, wobei die Auswahldaten, welche zulässige Adressenbereiche kennzeichnen, in einem entsprechenden Kennfeld festgehalten sind.

Bei paralleler Adressierung von die Signaldaten und die Auswahldaten enthaltenden Speichern und Verknüpfung über Block 301 kennzeichnen (beispielsweise durch eine über die Steuerebene 306 aufgerufene UND-Verknüpfung) die Auswahldaten nur solche Signaldaten als zulässige Adressen, welche einen von "Null" abweichenden Datenwert aufweisen. Der auswählbare Adressenbereich läßt sich also beliebig einschränken oder in sonstiger Weise beeinflussen. Bevorzugt wird hierzu eine beschränkte Anzahl von häufig zu verwendenden Verknüpfungsoperationen direkt über die Steuerebene aufrufbar vorrätig gehalten. Komplexeren Operationen können Eingangsgrößen auch durch einen entsprechend programmierten Kennfeldspeicher unterworfen werden, der jegliche Bewertung von Eingangssignalen durch entsprechende Vorgabe von Ausgangssignalwerten als Datenwerte in den betreffenden durch Kombination der Eingangssignale adressierten Speicherplätze zuläßt. Andererseits können Eingangssignale auch ausschließlich einer logischen Verknüpfung im Block 301 unterzogen werden, wobei das nachfolgende Kennfeld entweder bezüglich der Datenverarbeitung durch ein entsprechendes Steuersignal (nicht dargestellt) "überbrückt" wird oder aber es wird durch das betreffende Kennfeld dadurch unwirksam gemacht, daß in die Speicherplätze jeweils deren Adressen eingespeichert sind, so daß die Eingangssignale am Ausgang von Block 302 direkt wieder erscheinen.

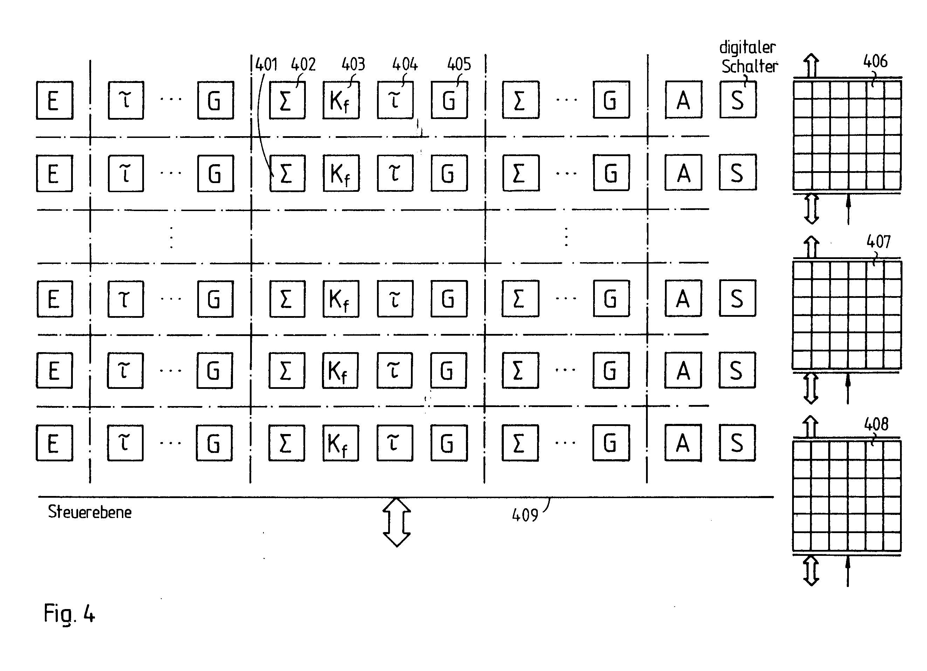

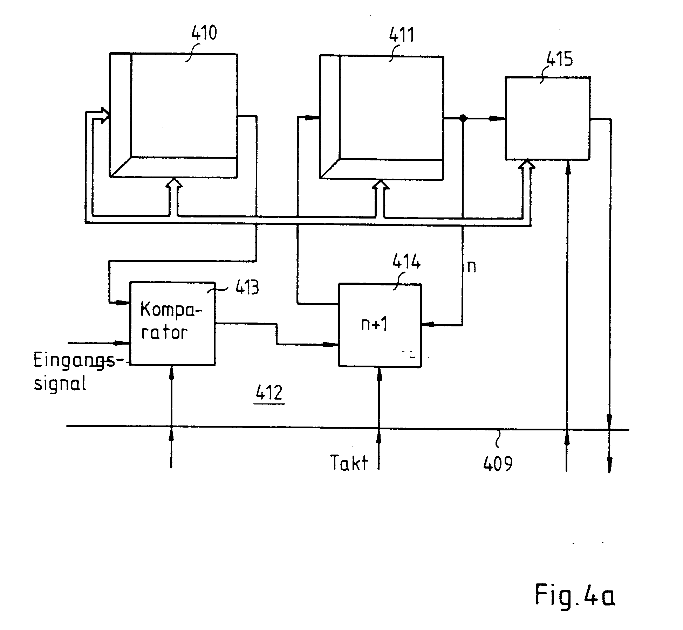

Die Steuerebene 306 selbst kann - wie auch anhand von Figur 4a weiter unten dargestellt ist - ebenfalls von Ausgangssignalen der Kennfeldspeicher 302 erreicht werden, so daß in Abhängigkeit von in Kennfeldern enthaltenen Signalen die Signalverknüpfung über die Blöcke 301 beeinflußbar ist. Ein in einem entsprechenden Speicher 302 enthaltener Datensatz enhält also neben einer Anweisung, ob es sich um Signal-, Auswahl- oder Verknüpfungs- bzw. sonstige Hilfsinformation handelt, im Falle der Auswahlinformation die Bezeichnungen der betreffenden Kennfeldzellen, die Ausgang und Eingang für das zu behandelnde Signal bilden. Bei Verknüpfungsinformationen kommt noch die vereinbarte Bezeichnung einer logischen oder mathematischen Operation hinzu.

Zusätzlich kann in Abhängigkeit von in Kennfeldern adressierten Daten auch mehrere Datenverknüpfungen in der Gesamtstruktur des Systems beeinflußt werden. Diese werden nämlich durch Verknüpfungsspeicher - wie weiter unten beschrieben - festgelegt. Wird durch ein in einem Speicherplatz enthaltenes Signal ein solcher Verknüpfungsspeicher aufgerufen, ändert sich die Systemstruktur mehr oder weniger grundlegend. Auf diese Weise ist der dargestellte Herzschrittmacher in der Weise "selbstlernend", daß sich die Konfiguration der Verarbeitung bezüglich der Meßwertauswahl und Verarbeitung weitgehend den vorgefundenen Eingangssignalen und deren Veränderungen selbsttätig anpassen kann. Derartige Veränderungen sind insbesondere: die Abschaltung von gerstörten Meßwertaufnehmern und dabei insbesondere der Übergang von einem durch mehrere physiologische Meßwertaufnehmer bestimmten Betrieb auf die durch einen bzw. keinen derartigen Aufnehmer beeinflußte Betriebsweise. Während zwei Meßwertaufnehmer für dieselbe Größe sich häufig im Differenzbetrieb betreiben lassen, kann ein einzelner verbleibender Aufnehmer häufig - bei entsprechend erhöhter Fehlerwahrscheinlichkeit - ebenfalls noch wirksam zur Steuerung beitragen.

Zusammen mit den Datensignalen werden bei der Bearbeitung noch Bewertungszahlen mitgeführt, die bei einer bewerteten Zusammenfassung eine Aussage über die Wertigkeit eines Eingangssignals bezüglich seiner erwarteten Zuverlässigkeit beinhalten. Die bewertete Zusammenfassung wird bevorzugt mit normierten Eingangssignalen vorgenommen. Sie verknüpft gleichartige Informationen enthaltende Eingangssignale jeweils zu einem zusammengefaßten Ausgangssignal, wobei dies nach den mathematischen Gesetzen für die Mittelwertbildung von Meßwerten mit Gewichtung erfolgt. Dabei kann diese Mittelwertbildung entweder durch digitale Verarbeitung über Errechnung nach einem formelmäßigen Zusammenhang erfolgen oder aber mittels einer abspeicherbaren Tabelle in Form des Rennfeldspeichers. Zur Ermittlung des Gewichts des betreffenden Eingangssignals werden zusätzliche Auswertungen des betreffenden Signals im Hinblick auf darin innewohnende Störungen, ausgedrückt durch nicht zu erwartende Schwankungen oder überlagerte Amplituden. Die entsprechenden Schaltungen zur Störerkennung entsprechen Schaltungen, wie sie bereits zur Elimination von gestörten Eingangssignalen in der Medizinelektronik angewendet werden.

Der Bagruppe 301 werden also digitalisierte Daten in verschiedener Art zugeführt. Im einzelnen werden dabei die Daten jeweils einer Eingangsebene nach einer über die äußere Programmierungs- und Zugriffsebene 306 vorgebbaren Verknüpfungen zusammengeführt. Die Eingangssignale $G_{11}$ bis $G_{1n}$ dienen zur Adressierung der Zeilen des Kennfeldspeichers 302, die Daten $G_1$ bis $G_3$ zur Adressierung der Zeilen, während die Daten 1 bis 3 die Dateninhalte - also die in den einzelnen adressierten Speicherplätze einzuschreibenden Daten betreffen.

Über die externe Steuer-(Zugriffs- bzw. Programmier)ebene ist die Art der Verknüpfung in dem Speicher 301 vorgebbar, wobei verschiedene mathematische Verknüpfungen (Summierung, Mutliplikation) oder logische Verknüpfungen auswählbar ist, so daß die Daten addiert, multipliziert oder jeweils nach einer logischen Bedingung überführbar sind, wobei die logischen Bedingungen außer UND-, UND/ODER oder weiteren bekannten, durch logische Gatter ausführbare Verknüpfungen auch aus "Größer", "Kleiner" oder "Gleich"-Beziehungen bestehen können. Dabei ist auch vorgebbar, daß beim Einhalten der vorgegebenen Bedingung ein bestimmter fester logischer Wert ausgegeben wird. Die Baugruppe 301 läßt also die Verarbeitung von Signalen sowohl mit festem logischem Pegel als auch solcher mit variablem Pegel in digitaler Darstellung zu.

Die Anzahl der Ebenen des Bauelementes 301 vergrößert sich entsprechend, wenn der Kennfeldspeicher 302 mehrdimensional ausgebildet ist, wie es durch die perspektivische Schichtung in Figur 3 angedeutet ist. Für jede Dimension ist eine zusätzliche Adressierungsebene erforderlich. Die Darstellung in Figur 3 hat dabei eine eher symbolische Form. Es ist jede andere zweckentsprechende Gestaltung der realen Speicher möglich.

Der Schaltung 302 ist eine programmierbare Signalverarbeitungsbaugruppe 303 nachgeschaltet, die eine zeitabhängige lineare Verarbeitung des Signalwerts ermöglicht, während in der Baugruppe 302 ein nichtlinearer tabellierbarer Zusammenhang zwischen Ein- und Ausgangsgröße als Kennfeld vorhanden ist. Die beiden Baugruppen 302 und 303 sind durch externe Programmiermittel über die Steuerebene zu beeinflussen, wobei bevorzugt in die Baugruppe 303 die Koeffizienten einer das zeitliche Übergangsverhalten bestimmenden linearen Differentialgleichung (in ebenfalls matrixartiger Anordnung entsprechend den übrigen Datenbaugruppen) eingebbar sind. Die zeitliche Verarbeitung erfolgt dabei durch den internen Mikroprozessor entsprechend solchen Systemen, die durch digital einstellbare Parameter die digitale Nachbildung linearer physikalischer Systeme ermöglichen. Durch die Trennung nichtlinearer und linearer Systemkomponenten der Signalverarbeitung ergibt sich bei einfacher Anpaßbarkeit eine einfache Systemstruktur, die extern unkompliziert einstellbar ist.

Der Signalverarbeitungsteil 303 kann auch eine Signalverzögerung in der Weise bewirken, daß mittels eines bestimmten Parameters eine vorgegebene Signalverzögerung (durch die Bezeichnung der entsprechenden Zahl von Taktsignalen eines Systemtakts) vorgegeben wird, wobei daraufhin die Ausgangssignale entsprechend der eingestellten Zahl nach Art einer digitalen Verzögerungsleitung (Schieberegister) erst um die angegebene Anzahl von Taktsignalen gegenüber dem Eingang verzögert am Ausgang erscheint. Die im Block 303 wiedergegebenen Darstellungen geben Beispiele für ein mögliches Übergangsverhalten dar, das durch die Kennwerte der betreffenden linearen Differentialgleichungen beschrieben wird.

Die Baugruppen 304 und 305 erzeugen eine Kennzeichnung für die Zuverlässigkeit des durch den Baustein 300 ausgebenen Signals, das dem Eingang der nachfolgenden entsprechenden Baustein zugeführt wird, so daß in jeder Stufe ein Maß für die Zuverlässigkeit gegeben ist, welches bei der nachfolgenden Zusammenführung berücksichtigt werden kann. Fehlerhafte Signale lassen sich auf diese Weise dadurch eliminieren, daß bei der nachfolgenden Verarbeitung statt dessen auf andere fehlerfreie Signale zurückgegriffen wird.

So kann ermittelt werden, ob der betreffende Eingang mit Störsignalen behaftet ist. Ein Anzeichen dafür ist dann gegeben, wenn bei Meßwerten, welche ihrer Natur nach eine langsame Veränderung unterliegen (Bluttemperatur etc.) Signale zur Verarbeitung gelangen, welche sprunghaften Charakter haben. Desweiteren ist ein Kriterium darin zu sehen, daß beim Vergleich zweier oder mehrerer Signale, die unter bestimmten Bedingungen ein gleichartigen Änderungsverlauf zeigen, Abweichungen von dieser Gleichartigkeit auftreten. Wichtig für die Verarbeitung bei dem dargestellten Schrittmacher ist die Tatsache, daß den digitalen Eingangssignalen in Form von einer zusätzlichen Information von mindestens einem Bit eine Kennzeichnung über die Zuverlässigkeit des entsprechenden Meßwerts - bzw. nach der zeitabhängigen Verarbeitung in einer Baugruppe 303 - für eine Verknüpfung von Meßwerten zugefügt wird.

Die Anordnung 304 zur Gewichtung enthält im einfachen Fall zur Störerkennung einen Mittelwertbildner und einen Vergleicher, dem das Eingangssignal von der Baugruppe 301 direkt und das Ausgangssignal der Störerkennung zugeführt wird, wobei bei Abweichungen, die über einen vorgegebenen Betrag hinausgehen, über das Ausgangssignal von 304 angezeigt wird, daß die Eingangssignale gegebenenfalls nur eine verringerte Zuverlässigkeit aufweist, so daß die Gewichtung bei der Bewertung entsprechend herabzusetzen ist. Das Signal G faßt die Gewichtungssignale von Vorstufen zusammen, die selbst schon eine entsprechene Bewertung vorgenommen haben. Es wird in der Baugruppe 305 dem der Stufe 304 zugefügt und die bewertete Zusammenfassung als Ausgangssignal der Stufe 300 zur Verfügung gestellt. Die Baugruppen 304 und 305 sind ebenfalls über die Steuerebene 306 sowohl abhängig von Signaldaten als auch durch äußere Programmierung - schaltbar.

Die Kennfelder enthaltenden "Zellen" können verschiedene Arten von Informationen enthalten. Sie bilden beispielsweise Festwertspeicher - auch ohne Verknüpfung ihrer Dateninhalte mit anderen Zellen, wobei sie durch physiologische Meßwerte oder Ausgangssignale anderer Kennwertspeicher adressierbar sind. Auf diese Weise lassen sich Bereiche, welche von den (Adressen bildenden) Eingangssignalen durchlaufen werden, für nachfolgende Stufen oder für die externe Kommunikation mit grafischer Darstellung besonders kennzeichnen. Zulässige Arbeitsbereiche oder hervorgehobene Bereiche sind vorgebbar oder aber logische Verknüpfungen. Wenn die Speicherinhalte als Ausgangssignale nicht für nachfolgenden Speicherzellen zur Adressierung oder als Datensignal bestimmt sind, können derartige Kennfelder auch lediglich zur Information des Benutzers oder zur Steuerung des Kommunikationssystems dienen, um bestimmte Betriebsbereiche für die externe Kommunikation bei den dort verwendeten Grafiken hervorzuheben.

In Figur 4 ist die Anordnung der zuvor beschriebenen Baugruppen in einer matrixartigen Struktur dargestellt, wie sie für die Adressierung der einzelnen Baugruppen im Rahmen ihrer Verknüpfung in einem Verknüpfungsspeicher 406 zugrundegelegt wird. Für die Zuordnung der Auswahl kann sie auch entsprechend grafisch dargeboten werden. Dabei umfaßt eine mittels Zeilen- und Spaltennummer adressierbare Funktionsgruppe jeweils die Funktionselemente, einer als solche durch Adressierung auswählbaren Zelle, bestehend aus Summierteil 402, Kennfeld 403, linearem Zeitglied 404 und Gewichtungseinheit 405 (entsprechend der Darstellung in Figur 3). Diese Funktionsgruppen wiederholen sich in einem beliebigen Zeilen-und Spaltenraster, wobei mittels zweier matrixartig organisierter Speicherbereiche einerseits für jede Funktionsbaugruppe bezüglich ihrer Eingänge das "Planquadrat" derjenigen Baugruppe eingebbar ist, deren Ausgangssignal dieser Baugruppe zugeführt wird. Da die Summiereinheit 402 mehrere Eingänge aufweist, können pro Baugruppe mehrere Spalten und Adressenkennzeichnungen vorgesehen werden.

Zusätzlich werden die ausgegebenen Signaldaten den nachfolgenden Zellen zugeordnet, wie es zuvor beschrieben wurde. In der äußersten rechten Spalte des die Verknüpfungsinformation aufnehmenden Speichers 406 sind beispielsweise die Eingangsgrößen des Schrittmachersystems jeweils einem Speicherelement einer Zeile zugeordnet, so daß durch die entsprechende Eingabe festgelegt werden kann, ob die Ausgangssignale welcher Baugruppen die betreffenden Parameter bilden sollen. Der erste Speicher 406 enthält die im Normalbetrieb gültigen Signalverknüpfungen - programmierbar durch den externen Kommunikationsteil.

In einen zweiten matrixartig organisierten Speicher 407 ist - jeweils wieder in Spalten- und Zeilenzuordnung - die Systemstruktur für einen alternativen Betriebszustand, wie er durch die Signaldaten selbst über die Steuerebene 306 aufrufbar ist, vorgegeben.

In Figur 4a ist dargestellt, wie durch Verknüpfung zweier Kennfeldzellen 410 und 411 (entsprechend den Zellen 401 in Figur 4) und einer Zählerzelle 412 eine Histogrammspeicher gebildet wird, kann, welcher beim späteren Auslesen Aufschluß über das Verhalten des Schrittmachersystems gibt.

Dabei bildet die Zelle 410 ein (programmierbares) Bezugsfeld, in dessen Speicherplätzen, welche durch Ausgangssignale vorangehender Zellen oder aber von Eingangssignalen adressierbar sind, Werte festgehalten werden, die als Bezugsgrößen bei Vergleichsoperationen dienen. In einer Zählermatrix 411 werden in den einzelnen Speicherplätzen, die ebenfalls matrixartig organisiert sind, jeweils Zählerstände festgehalten, die dem Auftreten bestimmter Ereignisse - im Normalfall dem Über- oder Unterschreiten des entsprechenden in der Zelle 410 enthaltenden Bezugswerts - festhalten. Die Speicherzellen 410 und 411 werden dazu parallel adressiert. Das Eingangssignal, welches mit den in der Bezugszelle 410 festgehaltenen Werten verglichen werden soll, wird einem Komparator 413 zugeführt, der es nach einer von außen vorgebbaren Bedingung mit dem jeweils adressierten Signal in der Bezugszelle 410 vergleicht. Ist die Bedingung erfüllt, so wird durch das einmalige Auftreten dieses Zustands (gesteuert durch ein Taktsignal) der am Ausgang des Zählspeichers in der adressierten Position vorhandene Wert um "1" erhöht und wieder eingelesen. Auf diese Weise ergibt sich ein später auszulesendes Kennfeld, welches in der graphischen Darstellung die Häufigkeit des Erreichens vorgegebener Betriebswerte übersichtlich anzeigt.

Weiterhin vorgesehen ist die Möglichkeit, das Betriebsverhalten des Schrittmachers auch unter Berücksichtigung der Häufigkeit des Einhaltens vorgegebener Betriebswerte zu verändern. Dazu wird vorzugsweise der Inhalt des Zählerkennfeldes 411 nach Figur 4a einem Block 300 gemäß Figur 3 als Eingangsgröße zugeführt und nach entsprechenden logischen Entscheidungen mittels eines entsprechenden Kennfeldes das Betriebsverhalten geändert. Dazu ist der aufgrund des Zählerstandes (Verhalten in der Vergangenheit) adressierten Kennfeld-Matrix ein weiterer Kennfeld-Speicher zum Aufruf von unterschiedlichen Kennfeldern nachgeordnet, welche vorhandene Verknüpfungen verändern (Speicher 406 in Figur 4, oder aber den Parameterspeicher (112 in Figur 1) beeinflussen bzw. als Festwertspeicher dienende Kennfelder entsprechend umschalten oder ersetzen. Dazu ist es günstig, wenn der Parameterspeicher 112, welcher das konventionelle Verhalten des Schrittmachers beeinflußt, ebenfalls entsprechend den Kennfeldern, die die physiologische Steuerung kontrollieren, aufgebaut ist. Auf diese Weise ist auch die Programmierung des konventionellen Teils entsprechend der Verarbeitung der physiologischen Parameter möglich.

"Physiologische" Meßgrößen, die im konventionellen Teil des Schrittmachers ermittelt werden, wie der Q-T-Abstand oder eine sich bei bestimmten Belastungen einstellende Spontanfrequenz, können somit über den Parameterspeicher 112 als Interface in das übrige System übertragen werden. Dieses Interface ist auch geeignet zur Übertragung bei der Ermittlung von physiologischen Größen über die Elektrode oder sonstige im konventionellen Bereich des Schrittmachers vorgesehene Meßwertaufnehmer.

Dem Ausgang des Blocks 411 (oder jeder anderen Baugruppe) ist durch über eine entsprechende Steuerung von der Steuerebene 306 ein Schaltelement nachschaltbar, welches in Abhängigkeit vom Ausgangssignal des Blocks 411 ein Signal zur Steuerebene abgibt, welches eines Verknüpfungsmatrix 406 beeinflußt, die ihrerseits wieder die Blöcke 301 (in Figur 3) ansteuert. Auf diese Weise läßt sich in Abhängigkeit vom - gegebenenfalls noch über vorgegebene Zeitabschnitte zu mittelnden - bisherigen Signalverhalten das zukünftige Verhalten beeinflussen. Es können Kennfelder umgeschaltet, d.h. ausgetauscht, unterschiedlich bewertet oder in ihrer Verknüpfung mit vorangehenden und nachfolgenden Kennfeldern verändert werden. Wie diese Veränderungen erfolgen sollen, wird in den beteiligten die Verknüpfungen festlegenden Kennfelder festgehalten. Das System wird somit ebenfalls "selbstlernend", wobei erst eine Anzahl von oder eine gewisse Häufigkeit von Ereignissen zur Änderung der Konfiguration führen. Maßgeblich für die Änderung des Systemverhaltens sind Störungen vorgegebener Häufigkeit oder Intensität oder auch das Nichteintreten vorbestimmter Betriebszustände, welche eine bestimmte Schrittmacherkonfiguration, von der Therapie her erforderlich machen, so daß die Stimulation stets in der einfachsten - und für den Arzt übersichtlichsten Betriebsweise erfolgen kann. Aus dem Speicher ist für den Arzt somit auch eine Erfolgskontrolle, der von ihm vorgenommenen Programmierung ersichtlich.

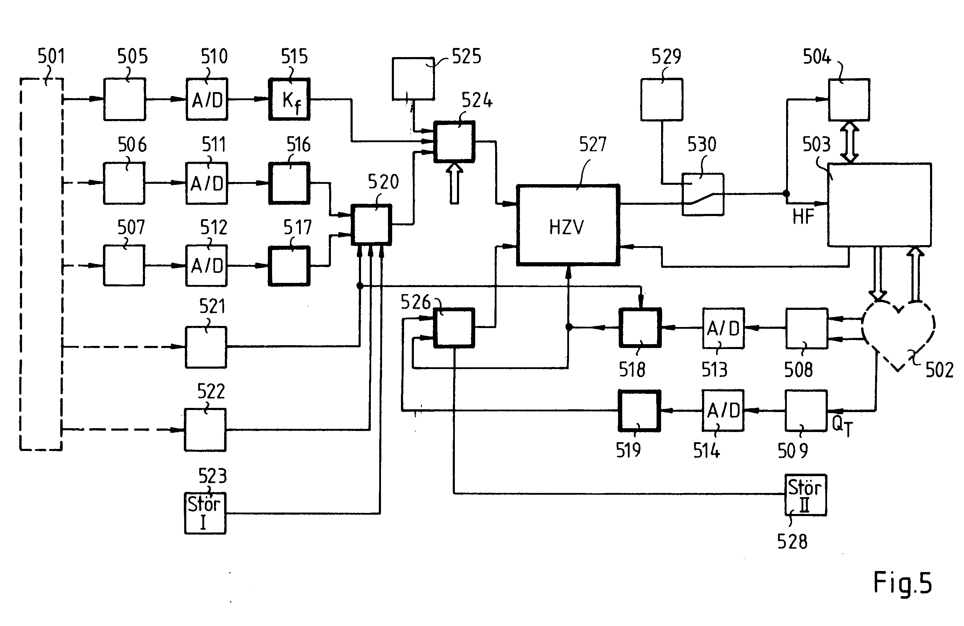

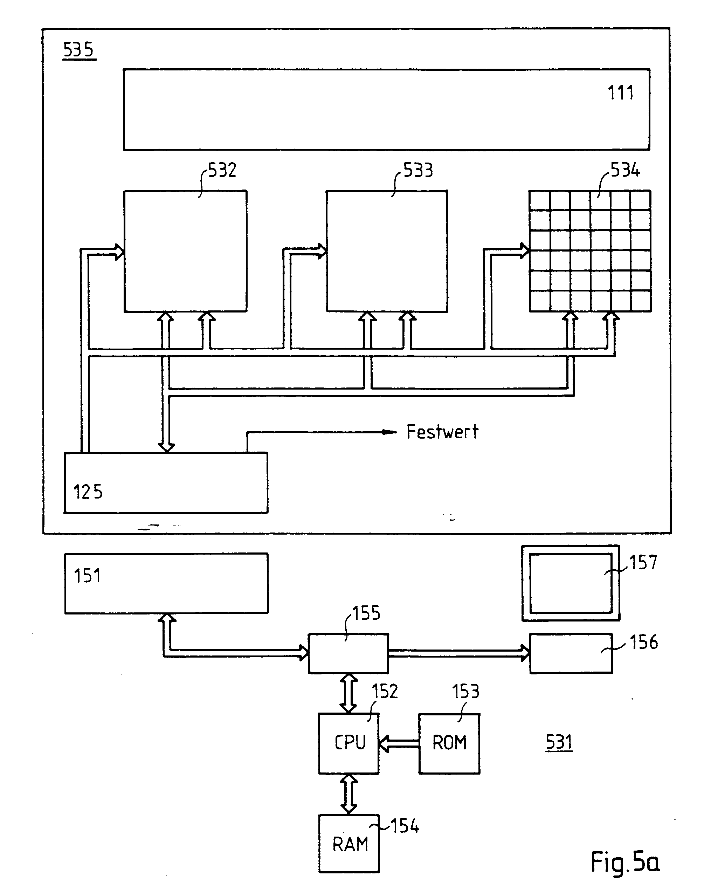

Nach der Charakterisierung der Grundbaugruppen soll nun das Zusammenwirken dieser Baugruppen im Blockschaltbild gemäß Figur 5 näher beschrieben werden. Insbesondere wichtig für den Betrieb des Systems ist, daß die Signalgrößen jeweils entsprechend ihrer physikalischen bzw. physiologischen Bedeutung verknüpft werden. Nach einer dem betreffenden Meßwertaufnehmer nachgeschalteten Eichtabelle (Kennfeld) erfolgt in einer weiteren Verarbeitungszelle eine Umwandlung in eine dem aktuellen Leistungsdefizit des Herzens angepaßte Größe. Der Bluttemperatur wird eine differenzierende Komponente zugeordnet, da die Erwärmung stets gegenüber der aktuellen Belastung verzögert erfolgt. Eine Belastungserhöhung oberhalb eines vorgegebenen Wertes wird zeitlich limitiert (Der Inhalt der Zählermatrix 411 in Figur 4a wird durch einen Systemtakt als Zeituhr heraufgesetzt.) Bei Überschreitung einer vorgegebenen Zeitdauer wird die ausgegebene Belastungsgröße durch eine entsprechende Umschaltung des betreffenden Kennfeldes über die Steuerebene erniedrigt, bis die Temperatur wieder einen niedrigeren Ausgangswert erreicht. Auf diese Weise wird bei einem Fieberzustand eine permanente Heraufsetzung der Herzfrequenz vermieden.

Die im Körper aufgenommenen physiologischen Größen werden jeweils auf entsprechenden "Verknüpfungsebenen" mit den weiteren gleichartigen Signalen verknüpft, so daß eine physikalisch korrekte Weiterverarbeitung erfolgt. Dabei wird zwischen zwei Verknüpfungsstufen (falls notwendig) jeweils eine Anpassung des zeitlichen und Amplitudenverhaltens des zu verarbeitenden und zu verknüpfenden Signals durchgeführt, so daß das betreffende Signal an der jeweiligen Verknüpfungsstelle mit einer weiteren physiologischen Größe, die bei der Steuerung der Herztätigkeit von Bedeutung ist, angepaßt ist. Damit ergibt sich der Vorteil, daß das System parallel zu den im Körper ablaufenden, eine Beeinflussung der Herztätigkeit hervorrufenden Signalen und körpereigenen Regelungsvorgängen arbeiten kann und in den verschiedenen Verknüpfungsstufen jeweils die betreffenden physiologischen Signale auf der betreffenden Stufe durch zusätzliche Meßwertaufnehmer ebenfalls erfaßt und entweder als Kontrollsignale benutzbar sind oder aber in die weitere Signalverarbeitung entsprechend ihrer Wertigkeit einfließen. Für die "Eichung" von Meßwertaufnehmern ist jeweils ein separater Kennfeldspeicher vorgesehen.

Dazu kommt, daß Signale, welche einerseits ein Maß für den Leistungsbedarf und andererseits mit kennzeichnend für das Herzzeitvolumen selbst sind sowie solche Größen, die nur kurzfristig zur Verfügung stehen (Kontrollmessung der körperlichen Belastung mittels eines Ergometers zwecks Eichung von Meßwertaufnehmern) ebenfalls systemrichtig eingespeist werden und eine Aussage über die Verarbeitungsfähigkeit der vorangehenden Stufen ermöglichen bzw. gestatten die entsprechenden Verarbeitungsparameter (Bausteine 302 und 303) richtig einzustellen. Ferner kann durch Rückführung einer Größe, welche einen Rückschluß auf die Herzleistungsfähigkeit zuläßt, ebenfalls die Signalverarbeitung beeinflußt werden. Diese Rückführung erfolgt dabei in der Weise, daß die physiologischen Meßwerte in der Verarbeitung zusammengefaßt und in der Weise umgeformt werden, daß sie ein Maß für den augenblicklichen Bedarf des Herzzeitvolumens bilden. Ein vom Herzen abgeleitetes Kriterium des tatsächlichen Herzzeitvolumens wird als Vergleichskriterium herangezogen, wobei dieses Herzzeitvolumen als Rückführung bei der Auswahl der Stimulationswerte im Rahmen einer Tabelle berücksichtigt wird. Bei einer bevorzugten Ausführung wird die die Stimulationsparameter einstellende Tabelle aufgrund des sich mit der Stimulation mit den betreffenden Parametern tatsächlich einstellenden Herzzeitvolumens aktualisiert. Dabei werden bei Eingangsadressen, welche eine bestimmte Herzleistung erfordern, diejenigen Stimulationsparameter oder Signalwerte, die zu den notwendigen Stimulationsparametern führen, eingetragen.

Für die Verknüpfung von Signalen in Form von digitalen Kennfeldern ergeben sich - entsprechend den genannten Anwendungen - folgende Grundprinzipien:

1. Reine Steuerfunktionen werden durch die einfache Verknüpfung von Eingangsmeßgrößen als Adressengrößen und die Stimulationsparameter als gespeicherte Werte gebildet.

2. Regelungsfunktionen werden in entsprechender Weise realisiert, wobei gemessene Eingangsgrößen durch entsprechende Kennfelder in eine Größe überführt werden, die für die körperliche Belastung, entsprechend dem erforderlichen Herzzeitvolumen repräsentativ ist. Diese Größe adressiert das Kennfeld zusammen mit einer für das aktuelle Schlagvolumen repräsentativen Größe, wobei die dann notwendige Herzrate aus den einzelnen Speicherplätzen auslesbar ist. Insbesondere ist auch statt der jeweiligen Absolutwerte die jeweilige Abweichung von einem vorgegebenen Sollwert als Signalwert verwendbar, wobei die nachfolgende Verarbeitung sich dann ebenfalls auf die entsprechenden Abweichungen bezieht. Bei Meßgrößen, welche mit einer zeitlichen Verzögerung dem tatsächlichen physikalischen Bezugswert folgen, wird eine Kompensation bevorzugt dort vorgesehen, wo die betreffende Meßgröße in möglichst unverfälschter Form vorliegt. Die zeitliche Verzögerung bei der Erwärmung des Blutes bei körperlicher Belastung wird durch eine differenzierende Komponente (303 in Figur 3) kompensiert, so daß die Ratenveränderung schneller eintritt.

3. Die Eichung einer insbesondere belastungsabhängigen Meßgröße erfolgt dadurch, daß im Eichzeitraum der erwartete (und gegebenenfalls auf einem anderen Meßweg festgestellte) Wert jeweils in den durch die aktuelle Meßgröße adressierten Speicherplatz einbeschrieben wird. Insbesondere wird dazu die extern mittels eines Ergometers ermittelte Belastung als in ein durch die für Belastung kennzeichnende(n) Meßgröß-(en) zu adressierenden Speicher jeweils als Wert einbeschrieben. Dieser Wert adressiert seinerseits während des nachfolgenden Betriebszustands in einem Kennfeld die entsprechende Rate, wobei diese Rate so gewählt ist, daß (insbesondere bei zusammengefaßter Adressierung mit einem für das aktuelle Schlagvolumen repräsentativen Meßwert) das Produkt aus Schlagvolumen und Rate als Herzzeitvolumen der ermittelten aktuellen Belastungsgröße entspricht und nachgeführt wird.

4. Die Fehlerkontrolle wird durch Vergleich von Zwei (oder mehr) Größen ermittelt. Das betreffende Kennfeld bewirkt bei über ein vorbestimmtes Maß hinausgehenden Abweichungen eine Abschaltung eines oder aller Werte. Eine Abschaltung des betreffenden Wertes erfolgt durch den Schalter 45 in Figur 4a durch Ausgabe eines den Schalter betätigenden Steuerwertes. Bei Adressierung von Speicherplätzen läßt sich entsprechend bei drei Vergleichssignalen dasjenige von der Weiterverarbeitung ausschließen, welches von zwei anderen wesentlich abweicht. Dazu ist ein Kennfeld erforderlich, welches durch Adressensignale adressiert wird, in denen alle drei Adressenkomponenten zusammengefaßt sind. Drei unterschiedliche als Abschaltbefehle wirkende Signale sind in den jeweils durch unzulässige Signalkombinationen adressierten Speichern enthalten und schließen bei einem abweichenden Signal dieses und bei zwei Abweichungen alle drei Signale von der Weiterverarbeitung aus. Außerdem sind (gegebenenfalls in Abhängigkeit von weiteren Signalen) Erwartungswerte vorgebbar, welche eine Weiterverarbeitung der Eingangssignale (durch logische Zuordnung in einem entsprechenden Kennfeldspeicher) nur dann zulassen, wenn das Eingangssignal in einem vorgegebenen Erwartungsintervall liegt.

5. In der Steuerebene ist eine ausgewählte Arbeitskennlinie (beispielsweise durch externe Kommunikation), derart vorgebbar, daß in einem zusätzlichen Kennfeld (durch nur eine Eingangsgröße adressierbar) der Wert der anderen Größe als Speicherinhalt fest zugeordnet wird. Das kann beispielsweise durch einen Eichvorgang (wie oben angegeben erfolgen). Das zweite Kennfeld ist dann nur "eindimensional". In einer perspektivischen (grafischen) Darstellung eines zweidimensionalen Kennfeldes lassen sich durch Überlagerung des eindimensionalen Kennfeldes die Punkte der Arbeitskennlinie auch grafisch hervorgehoben darstellen. Bei einem Arbeitsfeld wird ein entsprechender Bereich von Eingangssignalen in einem Kennfeld mit durch entsprechende Speicherinhalte und nachfolgenden logischen Verknüpfungen für zulässig erklärt und der Weiterverarbeitung zugeführt.

In Figur 5 ist ein Schrittmachersystem wiedergegeben, wie es sich dem behandelnden Arzt auf dem Bildschirm seines Steuergerätes in der Blockdarstellung darbietet. Es bildet gleichzeitig die Grundlage für die Funktionsbeschreibung eines Schrittmachersystems, wie es auch auf herkömmliche Weise - beispielsweise hardwaremäßig - erzeugt werden kann. Figur 5 gibt den implantierbaren Teil wieder, wobei die betreffende Anordnung selbstverständlich nicht auf implantierbare Systeme beschränkt ist, sondern auch entsprechend für externe Schrittmacher verwendbar ist. Der gestrichelt dargestellte Block 501 kennzeichnet die außerhalb des Herzens befindlichen Bereiche des menschlichen Körpers, von denen physiologische Meßgrößen hergeleitet werden, welche die Funktion des Schrittmachers betreffen, während das Herz 502 über die Stimulationselektroden und im Herzen plazierte Meßwertaufnehmer mit dem System in Wechselwirkung tritt.

Ein konventioneller Schrittmacher 503 ist autark funktionsfähig und gegebenenfalls über eine Programmiereinheit 504 multiprogrammierbar. (Bei einem gemäß Figur 1 dargestellten System, welches die realen Verknüpfungen wiedergibt, erfolgt die Programmierung durch die einheitliche Steuereinheit mittels des Parameterspeichers 112.)

In dem sich dem Arzt graphisch darbietenden System gemäß Figur 5 erfolgt die Programmierung mit einem Block 504, welcher auf dem Steuergerät als Seite aufrufbar ist und die konventionell programmierbaren Parameter in geläufiger Kennzeichnung enthält. Wahlweise lassen sich dabei unterschiedliche Typen von Schrittmachern komplett implementieren. Die Übersetzung der real in dem implantierbaren System eingestellten Parameter in ein konventionelles System erfolgt dabei im externen Steuerteil, wobei ein Wählschalter vorgesehen ist, welcher unterschiedliche Implementierungen erlaubt.

Bei den mit stark ausgezogenen Umrandungen versehenen Blöcken handelt es sich um Baugruppen gemäß Figur 4, welche die Funktionselemente gemäß Figuren 2 und 3 enthalten und mittels der in Figur 4 angegebenen Verknüpfungsmatrix in der dargestellten Weise logisch verbunden sind. Das Ausführungsbeispiel gemäß Figur 5 zeigt exemplarisch Beispiele für Verknüpfungen in einer bevorzugten Ausführung, wobei die Betriebsweise im einzelnen weiter unten wiedergegeben ist. Es ist ersichtlich, daß die Ausgangssignale verschiedener analoger Meßwertaufnehmer 505 bis 509 in der Blockdarstellung im Zuge der Signalverarbeitung jeweils in unterschiedlichen Gruppen verknüpfbar sind. Zuvor erfolgt eine Umsetzung der analogen Eingangsgrößen mittels Analog-/Digitalwandlern 510 bis 514. Die Ausgangsgrößen der Wandler werden jeweils Bewertungsbaugruppen 515 bis 519 zugeführt, wobei in diesen Baugruppen eine individuelle, d.h. programmierbare Anpassung erfolgt. Diese Anpassung schließt durch entsprechende Programmierung der zuvor dargestellten Kennfelder die Beseitigung von Nichtlinearitäten der Meßwertaufnehmer ein, ermöglicht die Einprogrammierung von zeitabhängigen Veränderungen der Übertragungscharakteristik sowie von Gewichtungsfaktoren. So läßt sich das Übertragungsverhalten eines Meßwertaufnehmers durch die Kennfeldprogrammierung verändern.

Während die Meßwertaufnehmer 506 den Sauerstoffpartialdruck im rechten Ventrikel oder die Respirationsrate erfassen, dient der Meßwertaufnehmer 507 der Ermittlung der Bluttemperatur, vorzugsweise in der Vena cava. Während der Sauerstoffpartialdruck oder die Respirationsrate ein gutes Maß für das augenblickliche Sauerstoffdefizit bilden - eine entsprechende Eichung des entsprechenden Kennfeldes vorausgesetzt - hat die Bluttemperatur integrierenden Charakter und steigt bzw. fällt erst mit einer gewissen Zeitverzögerung. Um in dem den Blöcken 516, 517 nachgeschalteten identischen Baustein 520, welcher die Ausgangsgrößen der vorangehenden Blöcke mit vorprogrammierter Gewichtung verknüpft, (wobei als übereinstimmende Bezugsbasis eine Belastungsgröße erhalten wird) ist innerhalb des Bausteins 517 die Zeitkonstante durch entsprechende Programmierung mit einer differenzierenden Charakteristik versehen, so daß vorzugsweise die differentiellen Änderungen der Bluttemperatur als Maß für einen Aktivitäts- oder Ruhezustand mit der Ausgangsgröße des Blocks 516 verknüpft sind.

Die Überlagerung kann entweder durch gewichtete Summenbildung linear oder aber mittels eines Kennfeldes erfolgen, wobei bei einem zweidimensionalen Kennfeld je eine Eingangsgröße eine Koordinatenachse adressiert. Die Verknüpfung im Baustein 520 ist ferner abhängig von einer Anzahl digitaler Ausgangssignale abgebender Sensoren 521 bis 523, welche - wie zuvor anhand von Figur 2 beschrieben - Umschaltungen des im Block 520 vorhandenen Kennfeldes bewirken. So erkennt ein Quecksilberschalter 521, welcher mit dem Schrittmachergehäuse implantiert ist, die augenblickliche Position des Patienten (liegend, stehend) und liefert damit eine zusätzliche Bewertungsmöglichkeit für die Belastung des Patienten. Ein digitaler Aktivitätssensor 522 erkennt darüber hinaus durch Auftreten von Beschleunigungen und Verzögerungen oberhalb eines vorgegebenen Grenzwertes, ob der Patient sich zur Zeit generell in Ruhe oder in Bewegung befindet und schaltet entsprechend das im Block 520 befindliche Kennfeld um. Die Steuerung der Meßwertverarbeitung erfolgt durch einen gemeinsamen Systemtakt, um die Synchronität sicherzustellen.

Eine digital arbeitende Störerkennung erfaßt, ob eine Belastungsgröße zur Zeit gerade nicht ermittelt werden kann. So werden beispielsweise, wenn beispielsweise die Respirationsrate mittels eines Mikrofons aufgenommen wird, durch ein innerhalb der Störerkennungseinheit 523 vorhandenes zusätzliches Mikrofon starke Geräusche, die durch ein Husten des Patienten oder dergleichen hervorgerufen werden, erkannt und zur Austastung der Meßwerte des Aufnehmers 506 benutzt, was durch Umschaltung des entsprechenden Kennfeldes oder aber auch durch Herabsetzung des Gewichtungsfaktors durch Verbindung mit dem Block 516 erfolgen könnte.

Ein zusätzlicher Meßwertaufnehmer 505 bildet eine Erfassung für die Aktivität des Patienten mit analogem Ausgangssignal. Entsprechend der Baugruppe 522 werden die Beschleunigungen und Verzögerungen nach Amplitude und Häufigkeit erfaßt und über die Baugruppen 510 und 515 weiterverarbeitet.

Während in der Baugruppe 520 die im Kreislaufsystem erfaßten Belastungsgrößen zusammengefaßt wurden, dient der Block 524 der Zusammenführung der Ausgangsgröße des Blocks 520 mit einem die tatsächliche physische Aktivität bezeichnenden Signal. Das Ausgangssignal des Blocks 524 gibt damit den Herzzeitvolumen(HZV)-Bedarf an. Die Ausgangssignale der Blöcke 514 und 520 sind dabei teilweise redundant und können zur Erhöhung der Zuverlässigkeit im nachfolgenden Kennfeld miteinander in Beziehung gesetzt werden.

Von besonderer Bedeutung ist ein zusätzlicher Übertragungsbaustein 525, welcher in das Telemetriesystem gemäß Figur 1 einbezogen ist. Über diese Baugruppe lassen sich externe mittels eines Ergometers ermittelte Belastungsdaten in dem Block 525 einspeisen, wobei durch externe Programmierung sowohl die Ausgangsgröße des Blocks 515 als auch die des Blocks 520 mit der augenblicklichen Last in Beziehung gesetzt werden. Durch Einsetzen der aktuellen Belastungsdaten in die mittels der Ausgangssignale der Blöcke 515 bzw. 520 adressierten Speicherplätzen des Kennfelds in Block 525 lassen sich die anderen den HZV-Bedarf kennzeichnenden Signale kontrollieren bzw. eichen, so daß die Zuverlässigkeit des Systems erhöht oder eine Regelung erzeugt wird. Durch das Übertragen und Abgleichen des außerhalb des Körpers verfügbaren Belastungssignals und Abgleich mit dem entsprechenden körperintern erhaltenen Signals ergibt sich eine Eichmöglichkeit oder Regelverknüpfung.

Zwei weitere Meßwertaufnehmer 508 und 509 erfassen Signale, welche ebenfalls das Herzzeitvolumen betreffen. In der Darstellung gemäß Figur 5 bildet der Meßwertaufnehmer 508 für das Schlagvolumen (über die systolischen Intervalle als Drukkaufnehmer oder Mikrofon bzw. durch Impedanzkardiografie oder Kombination beider Verfahren) ein Maß für die Volumendadaption des Herzens als Reaktion auf eine vorgegebene körperliche Belastung. Der weitere Meßwertaufnehmer 509 für den QT-Abstand ermittelt aus den im Herzen an den Stimulationsimpulsen aufgenommenen Signalen ebenfalls mittels eines entsprechenden Kennfeldes ein Maß für den aktuellen Frequenzbedarf. Nach einer entsprechenden Analog-Digital-Wandlung in Wandlerblöcken 513 bzw. 514 und eine gegebenenfalls nichtlinearen Verzerrung des Übertragungsverhaltens in Kennfeldgruppen enthaltenden Blöcken 518 und 519 folgt die Zusammenfassung der die Frequenz bestimmenden Größen in einem Block 526.

Die Verknüpfung der einen HZV-Bedarf kennzeichnenden Größen erfolgt im Block 527, wobei je nach Programmierung des Blocks 527 verschiedene Verknüpfungen gewählt werden können: Entweder werden die Ausgangsgrößen der Blöcke 524 und 526 einander überlagert und steuern die Herzfrequenz (als Grundrate des Demand-Schrittmachers 503) direkt. Diese Steuerung kann unter Berücksichtigung des augenblicklichen Schlagvolumens (Pfeil von Block 518 nach 527) vorgenommen werden, so daß alle diejenigen Größen, welche auf die Herzrate Einfluß haben, zusammengefaßt verarbeitet werden und sich ein den augenblicklichen aufgrund der ermittelten Größen günstiges Herzzeitvolumen einstellt. Die zusätzliche Bewertung des Schlagvolumens in Block 527 kann mit einer anderen Gewichtung erfolgen als diese im Block 526 als Tendenz für die einzuhaltende Frequenz eingegeben wird, so daß hier insbesondere Regelschwingungen vermieden werden. Wenn eine Vielzahl stabil zu erfassender Größen vorhanden ist, kann gegebenenfalls das Schlagvolumen bei der Verarbeitung im Block 526 auch ganz fortgelassen werden. Entsprechends gilt für die Größe $Q_t$.

In entsprechender Weise kann der Schrittmacher auch im geregelten Betrieb benutzt werden, wobei lediglich die Verknüpfung im Baustein 527 umzustellen ist. Hierzu wird der HZV-Bedarf im Kennfeld gemäß Block 527 abgeglichen mit dem Produkt aus dem Schlagvolumen und der augenblicklichen Rate, wobei die Rate heraufgesetzt oder erniedrigt wird, bis das Produkt von Schlagvolumen und -frequenz dem durch den Block 524 vorgegebenen Herzzeitvolumen entspricht. Es ist ersichtlich, daß durch unterschiedliche Verknüpfungen verschiedene Steuer- oder Regelmechanismen realisiert werden können, wobei insbesondere innerhalb des Kennfeldes die Rate mittels einer Suchstrategie schrittweise so variiert werden kann, daß ein möglichst großes Zeitvolumen erreicht wird.

Eine zusätzliche Störerkennung 528 schaltet die im Herzen aufgenommenen Signale ab, wenn dort Störungen erfaßt sind, wobei die Störkriterien denjenigen entsprechen, welche bei Demand-Schrittmachern die Steuerung des Schrittmachers vom Herzen unterbinden.

Ein Pfeil vom Ausgang des Blockes 527, welcher zum Parameterspeicher 504 gerichtet ist, zeigt an, daß die programmierten Parameter des Schrittmachers verändert werden. Dazu gehört insbesondere eine Dehnung der programmierten Zeitgrößen, wie Refraktär- oder Blanking-Zeiten mit abnehmender Frequenz. Dieser Zusammenhang läßt sich generell auf die Zeitsteuerung des Schrittmachers beziehen. Die programmierbare Veränderung in dem Block 504 erfolgt dabei vorzugsweise mittels eine dort gespeicherten Kennfelds, dessen die Programmierten Betriebswerte des Schrittmachers enthaltenden Speicher durch eine von der Frequenz hergeleitete Größe adressiert werden.

Bei dem Schrittmacher 503 handelt es sich insbesondere um einen Einkammerschrittmacher für die ventrikuläre Stimulation, da auf diese Weise das Legen einer zusätzlichen Elektrode ins Atrium entfällt. Die Meßwertaufnehmer für weitere physiologische Größen sind dabei im Schrittmachergehäuse oder in der Ventrikelelektrode angeordnet, so daß die Implantationstechnik sich nicht von der herkömmlicher Schrittmachern unterscheidet bzw. gegenüber AV-Schrittmachern sogar vereinfacht ist.

In Figur 5a ist dargestellt, wie durch die externe Kommunikationseinheit die einzelnen Kennfelder 531 bis 534, wie sie in den entsprechenden Zellen - wie zuvor dargestellt - adressiert und dort befindliche Daten ausgelesen bzw. neue Daten eingelesen werden. Die Adressierung erfolgt unabhängig vom übrigen Betrieb des Herzschrittmachers 535, welcher durch nicht physiologische Daten bestimmt ist, wobei während der Programmierung des physiologischen Teils diese Steuerung inaktiv geschaltet wird (Signal Festwert). Die Stimulationsgrundrate und die übrigen physiologisch bestimmten Betriebsparameter werden während der Programmierung auf einen dem Ruhezustand des Patienten entsprechenden Wert festgelegt.

Als Besonderheit ist weiterhin vorgesehen, daß durch die im Schrittmacher enthaltenen Konfigurationsdaten (Kennfeld 534) welche die datenmäßige Verknüpfung der Kennfelder festlegen, eine Graphikdarstellung abgerufen wird, die dieser Konfiguration entspricht. Dabei sind die einzelnen Kennfelder als Blöcke auf einem Bildschirm oder LCD-

Schirm wiedergeben und können durch Anwählen mit einem Lichtgriffel oder im Falle eines berührungsempfindlichen Displays mittels Druck zur Programmierung aufgerufen werden. Das jeweils aufgerufene Kennfeld oder der entsprechende Baustein erscheint dabei vergrößert auf dem Bildschirm, so daß die mit einem Cursor gekennzeichneten Datenfelder verändert werden können. Zur Erleichterung des Programmierens können in sogenannter "Fenster"-Technik beliebige Einzelstrukturen des Verknüpfungsdiagramms aufgerufen und verändert werden. Dabei können nicht nur Datensätze, sondern je nach Zugangsberechtigung des Kommunikationsteils auch Konfigurationen der Verarbeitung (Ein- und Ausgangsverknüpfungen) verändert werden, so daß das System äußerst flexibel ist und nach den gewonnen Erfahrungswerten patientenindividuell abgestimmt werden kann. Zum Aufrufen der graphischen Darstellung der Bildschirmkonfiguration werden entweder die Daten des Konfigurationskennfeldes zur direkten Adressierung eines entsprechenden Speicherbereiches verwendet oder aber auf Grund der betreffenden Verknüpfungsinformationen wird in CAD-Technik ein entsprechenes Blockschaltbild synthetisiert.

Besonders vorteilhaft ist dabei, daß das dargestellte Kommunikationssystem in ein standardmäßiges Datenverarbeitungssystem (PC oder ähnliches) einfügbar ist, wobei die Kommunikationsschnittstelle zum Schrittmacher eine zusätzliche in den PC einsteckbare bestückte Leiterplatte bildet, die mit der zugehörigen die vorgenannten Funktionen programmiert enthaltenden Software geliefert wird, so daß das Kommunikationsteil bei vorhandenem PC nur geringe Merkosten erfordert. Trotzdem ist - entsprechend dem vorhandenen Ausbau des PC - eine sehr große Verarbeitungskapazität vorhanden, welche den simultanen Aufruf und die Darstellung auch komplexer Darstellungen ermöglicht, wobei die Bedienerführung dem Anwender für die Konfigurationenprogrammierung des Systems Hinweise und Warnungen gibt. Über einen Drucker wird ein Protokoll über den Programmiervorgang erstellt, welches den eingestellten Betriebszustand des Schrittmachers dokumentiert. Die programmierten Dateneingaben bzw. die Konfiguration kann gleichzeitig in einen zentralen Speicher abgelegt werden, so daß die Betriebsparameter des betreffenden Schrittmachers bevorzugt auch über externe Datenkommunikationsmöglichkeiten abrufbar sind und in Notfällen jedem Arzt zur Verfügung stehen. Der Programmierteil ist dabei insbesondere auch als Kommunikationsterminal ausgelegt, so daß komplexe Software, welche auch die Bedienerführung und die Konfiguration des Schrittmachers betrifft, zentral gespeichert ist und dem jeweiligen Kommunikationsterminal übermittel wird. Auf diese Weise ist sichergestellt, daß jeweils bei der Programmierung die neueste Software verwendet wird, so daß eine Weiterentwicklung - unter Berücksichtigung des jeweiligen Hardwarezustands - auch nach der Implantation erfolgen kann. Die Erfahrungen mit einem bestimmten Schrittmachertyp an einer Vielzahl von Patienten mit entsprechenden Krankheitsbildern tragen also nachträglich gegebenenfalls zur weiterer Entwicklung bzw. Verbesserung des bereits implantierten Systems bei.

Das auf dem Bildschirm der Kommunikationseinhiet wiedergegebene Darstellung entspricht beispielsweise einem Blockdiagramm, wie es in Figur 5 wiedergegeben ist.

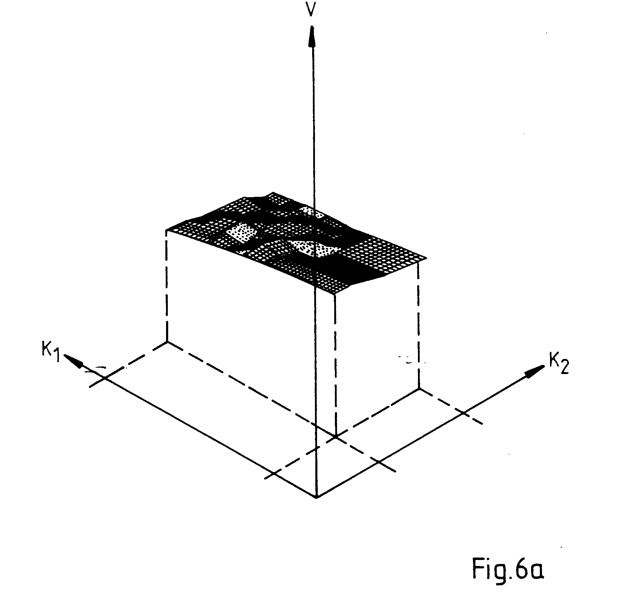

Die in den Figuren 6a bis 6c wiedergegebenen Kennfelder geben Beispiele für in matrixartig organisierten Speichern abgelegte Zusammenhänge zwischen Größen wieder, wie sie beim vorstehend beschriebenen Schrittmacherkonzept verwendet werden. Die Darstellung erfolgt bevorzugt in dreiachsigen Koordinatensystemen, damit eine perspektivische grafische Wiedergabe im externen Steuergerät erfolgen kann.

Bei der Ausnutzung nur zweier Koordinatenachsen erfolgt die Darstellung in der Ebene. Eine zusätzliche Darstellungsmöglichkeit besteht durch Überlagerung zweier Kennfelder in einem Diagramm. Zur Vereinheitlichung der Ansteuerung basieren die hier gewählten Kennfelder auf dreiachsigen Systemen, da der dazugehörige Speicher dann als zweidimensionale Matrix aufgebaut ist, so daß die entsprechenden Zahlenwerte ebenfalls in der Ebene, d.h. im Bildschirm darstellbar sind und somit also entweder durch grafische Eingaben mittels Lichtgriffel oder aber durch Zahleneingabe mit Cursor-Adressierung veränderbar sind.

Bei dem in Figur 6a wiedergegebenen Kennfeld können die Zahlenwerte K1 und K2 zwei verschiedene Eingangsgrößen sein, die verknüpft werden oder aber es handelt sich um eine Meßgröße und einen Parameter - beispielsweise die aktuelle Herzfrequenz bzw. die Bluttemperatur. Somit kann eine nichtlineare Kennlinie eines Meßwertaufnehmers mit Temperaturkorrektur durchgeführt werden.

Durch Überlagerung zweier Kennfelder läßt sich eine Eichung, in dem die beiden Kennfelder räumlich aneinander angeglichen werden oder aber es läßt sich eine Regelung einer vorgegebenen Strategie realisieren, welche beispielsweise darin besteht, daß das Volumen zwischen zwei überlagerten Kennfeldern gemacht wird oder aber die abhängige Veränderliche durch schrittweise Variation einer unabhängigen Veränderlichen ein relatives oder absolutes Maximum gesucht wird. Im letztgenannten Fall stellt die unabhängige Veränderliche einen Meßwert dar, wobei die abhängigen Veränderlichen entsprechend der Strategie variiert werden und nach einem Differenzkriterium die Veränderung überwacht wird.

Bei einer weiteren Ausführung bildet eine der Achsen die Zeitachse, so daß mittels der Kennfelder Herzereignisse der Vergangenheit, insbesondere als Histogramme, gespeichert und mit den selben grafischen Darstellungsmitteln aus dem Herzen heraus zum Steuergerät übertragbar sind, welche auch das übrige Schrittmachersystem betreffen.

Entsprechend ist im Steuerteil mit grafischen Mitteln das zeitabhängige Verhalten des Schrittmachers in Abhängigkeit von Herzereignissen darstellbar, wie es von herkömmlichen Schrittmachern aus der Darstellung der Stimulationsrate in Abhängigkeit der Folgefrequenz von spontanen Herzaktionen bekannt ist. Während bei den Steuerfunktionen die Kennfelder also fest programmiert sind, dienen sie bei der Regelung als Speicher für die veränderlichen Werte, welche als Grundlage für das Regelkriterium dienen, wobei der Regelvorgang durch grafische Darstellung des aktuellen Feldes der zuletzt durchlaufenen Werte für den behandelnden Arzt den Zustand des Systems grafisch anschaulich macht. Durch entsprechende Programmierung ist das System - wie ausgeführt - jederzeit zwischen einem Steuerungs- und einem regelnden System schaltbar. Diese Umschaltung kann in Abhängigkeit von vorangehenden Signalen selbsttätig erfolgen, wenn eine entsprechende Kennfeldzuordnung vorgesehen ist.

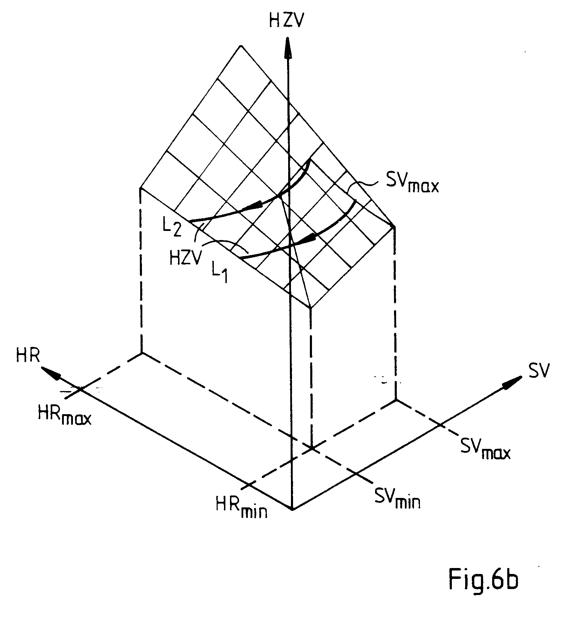

In Figur 6b ist in einem weiteren Kennfeld das Herzzeitvolumen in Abhängigkeit von der Herzrate und im Schlagvolumen angegeben. Dieses Kennfeld hat für die Steuerung des Schrittmachers besondere Bedeutung, da das Herzzeitvolumen ein Maß für die Leistungsfähigkeit des Herzens ist, zumal die Veränderung der Herzrate für sich allein kein ausreichendes Kriterium darstellt, wenn das erzielbare Schlagvolumen außer Betracht gelassen wird. Das in Figur 6b wiedergegebene Kennfeld zeigt den rein mathematischen Zusammenhang, der sich rechnerisch ergibt, wobei zwei verschiedene Lastkurven L1 und L2 eingezeichnet sind, unter der Voraussetzung, daß einer konstanten körperlichen Last bei der Steuerung des Schrittmachers ein ebenfalls konstantes entsprechendes Herzzeitvolumen entgegenzusetzen ist. Das Schlagvolumen ist zwischen dem minimalen und maximalen physiologisch zulässigen Werten veränderbar dargestellt, wobei entsprechendes für die Herzrate gilt. Beim Schrittmacher läßt sich jedoch nur die Herzrate beeinflussen, während das Schlagvolumen entsprechend der vorhandenen Adaptionsfähigkeit des Herzens selbsttätig einstellt. Es liefert also einerseits als Produkt mit der Herzrate ein Maß für die aktuelle Leistung des Herzens, während andererseits eine Verkleinerung oder Vergrößerung - bezogen auf eine konstante Herzrate - ein Kriterium dahingehend bildet, daß das körperinterne Regelsystem eine Vergrößerung oder Verkleinerung des Herzzeitvolumens wünscht, soweit diese Adaptionsfähigkeit bei dem betreffenden Patienten noch vorhanden ist.

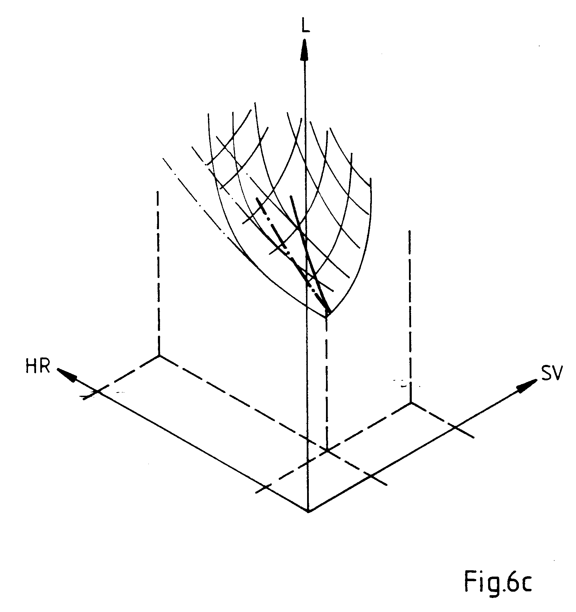

Um dem Arzt eine bessere Beurteilung dahingehend zu ermöglichen, welche Folge die einzelnen Steuer- oder Regelgrößen und gegebenenfalls eine entsprechend gewählte Arbeitskennlinie der Steuerung haben, dient die Darstellung gemäß Figur 6c. Hier ist in einem weiteren Kennfeld die Abhängigkeit des Schlagvolumens von der Herzrate und der Belastung wiedergegeben, wobei die axiale Richtung entsprechend dem im Kennfeld gemäß Figur 6b gewählt wurde, um einen vereinfachten Vergleich zu ermöglichen. Dabei zeigt sich, daß das Schlagvolumen bei - wie hier wiedergegeben - beschränkter Adaptionsfähigkeit des Herzens zu höheren Werten hin beschränkt ist, wobei zu höheren Frequenzwerten sich das Schlagvolumen verkleinert, weil die Kammerfüllung sich insbesondere beim ischämischen Herzen verringert. Durch Überlagern der Werte entsprechend der beiden Diagramme 5b und 5c ist der Arbeitsbereich des Schrittmachers festzulegen, wobei bei der selbsttätigen Einstellung durch Kennfeldüberlagerung davon ausgegangen wird, daß die äußere Belastungsgröße nicht um mehr als einen vorgegebenen Betrag vom Herzzeitvolumen abweichen darf, wenn das Leistungsvermögen des Herzens der Belastung längerfristig adäquat sein soll.

Es zeigt sich in der durchgezogenen Darstellung, daß eine zu starke Heraufsetzung der Herzfrequenz (Kennlinie mit geringer Steigung in bezug auf Achse HR) zu einem Absinken des Herzzeitvolumens führt und daher im dargestellten Fall nicht günstig ist. Günstig ist dagegen der eingezeichnete Verlauf einer Kennlinie, wobei die Belastung des betreffenden Patienten wegen der eingeschränkten Volumenadaption des Herzens beschränkt bleiben muß. Verläuft das Kennfeld gemäß Figur 6c dagegen zu höheren Frequenzen HR hin - wie gestrichelt dargestellt - so kann die Arbeitskennlinie flacher gewählt werden zu höheren Herzfrequenzen hin ist ein Bereich ohne Verringerung des Schlagvolumens erreichbar, bei dem das Herzzeitvolumen entsprechend vergrößert ist. Sind Zeiten mit Spontanaktivität des Herzens vorhanden, so wird die Arbeitskennlinie gemäß Figur 6c durch ergometrische Belastung des Patienten während Spontanaktionen ermittelt und im Stimulationsfall unter Benutzung des Schlagvolumens als Steuergröße entsprechend benutzt werden.

Im Falle der zusätzlichen Kenntnis von Meßgrößen, welche - wie beschrieben - den Bedarf des Herzzeitvolumens aufgrund der aktuellen physiologischen Belastungsdaten des Herzens ermöglichen, ist eine weitere Möglichkeit der Steuerung gegeben, wobei in diesem Fall die sich aufgrund des sich einstellenden Schlagvolumens im Diagramm 6c und die aufgrund des benötigten Herzzeitvolumens gemäß Figur 6b sich ergebenden Arbeitspunkte überlagert und eine Mittelung aufgrund der gewichteten Daten vorgenommen wird, wobei jedoch ein zulässiger Frequenzbereich vorgebbar ist, welcher sich aus dem Vergleich der Diagramme gemäß Figuren 6b und 6c unter den genannten Bedingung ergibt, daß das Herzzeitvolumen und die Belastung im Kennfeld nicht wesentlich voneinander abweichen sollten.

Die Kennlinie gemäß Figur 6c wird insbesondere so gelegt, daß Frequenzbereiche, in denen zunehmende Last eine Verringerung des Schlagvolumens (in bezug zu dem entsprechenden Schlagvolumenwert bei höherer Frequenz) umgangen werden, da in einem solchen Fall ein höherer Frequenzwert zu einer besseren Herzleistung führt. Diese Zusammenhänge sind aber bei der Kennfelddarstellung ohne weiteres anschaulich und der behandelnde Arzt kann durch die Verwendung des Steuergerätes mit grafischer Darstellungsmöglichkeit optimale Zuordnungen treffen.

Eine zusätzliche günstige Anwendung der hier dargestellten Kennfelder besteht in der grafischen Kontrolle der Anpassung der programmierbaren Zeitkonstanten. Dabei werden solche Meßgrößen, die mit Zeitkonstanten behaftet sind - wie beispielsweise die Thermoregulation des Kreislaufs unter Berücksichtigung des Blutvolumens. In diesem Fall wird bei einer externen sprungartig ansteigenden Belastung der Anstieg der Bluttemperatur durch Vergleichsmessung kontrolliert und zeitlich dargestellt, und die in der entsprechenden Verarbeitungsbaugruppe enthaltende "programmierbare Zeitkonstante" wird jetzt so variiert, daß sich der daraus ergebende theoretische Verlauf dem tatsächlichen Zeitverhalten möglichst angepaßt ist, wobei in der dreiachsigen perspektivischen Kennfelddarstellung die Variation eines Parameters als zusätzliche unabhängige Veränderlich berücksichtigt werden kann.

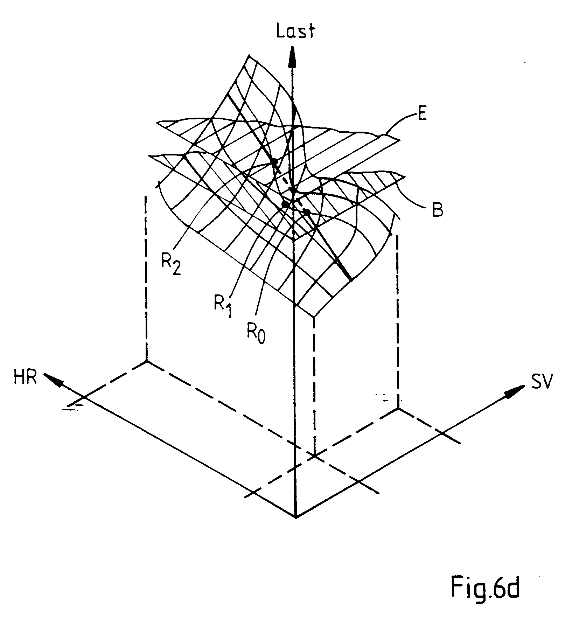

In Figur 6d wird anhand des aus den vorigen Figuren bekannten Diagramms die Verknüpfung der Steuerung der Herzrate durch mehrere Eingangssignale nach einer ExklusivODER-Verknüpfung dargestellt. Die Darstellung ist gleichzeitig ein Beispiel für eine Systemkonfiguration, bei der der Signalverarbeitungsweg von einem Signalereignis selbst abhängig ist. Dabei erfolgt die Beeinflussung der Herzrate zunächst nach einer mit dem Schlagvolumen verknüpften Weise, wie es zuvor dargestellt ist. Bei dem im Diagramm dargestellten Pfeil wird aber davon ausgegangen, daß die Veränderung des Schlagvolumens bei den betreffenden Patienten (oder eine entsprechende Größe) nur eingeschränkt zur Herzfrequenzsteuerung heranziehbar ist.

Bei der Rate $R_1$ sei ein Einbruch I vorhanden, welcher dazu führt, daß die Belastung B im Randbereich des nutzbaren Feldes ansteigt, ohne das die Rate $R_1$ das Schlagvolumen auf einen dieser Belastung adäquaten Wert heraufgesetzt wird. Bei einer ausschließlich vom Schlagvolumen beeinflußten Herzrate würde die Rate einen maximalen Wert $R_0$ erreichen und würde sich bei Vergrößerung der Belastung wieder verkleinern, da das Schlagvolumen zu der Frequenz $R_1$ hin einen Einbruch erleidet und sich von $R_0$ an verkleinert, was wieder zur einer Ratenverringerung führt. $R_0$ bildet also in diesem Fall die obere Ratenbegrenzung des Schrittmachers.

Um auch in diesem Fall den Patienten eine physiologisch korrekte Steuerung der Rate zu bieten, wird zusätzlich ein weiterer lastbezogener Parameter des Schrittmachers (beispielsweise die Bluttemperatur) zur Steuerung herangezogen. Die aufgenommene Bluttemperatur wird in einem entsprechenden Kennfeld in das zugehörige Belastungsniveau B umgesetzt, welches mit dem Ausgangssignal des Kennfeldes, welches die Rate in Abhängigkeit von einer mit dem Schlagvolumen korrelierten Größe programiert enthält, mittels einer ODER-Beziehung verknüpft, so daß die Steuerung der Herzrate von der Bluttemperatur übernommen wird, wenn beim Erreichen eines bestimmten Temperaturniveaus eine zugeordnete Herzrate nicht erreicht wird. In der zur Überwachung für den Arzt dargebotenen graphischen Darstellung wird Belastungsniveau in Abhängigkeit von der Temperatur durch eine horizontale Fläche dargestellt, welches sich pegelartig vertikal verschiebt. Überschreitet das Niveau einen Belastungswert, der größer ist als eine entsprechende Belastung, die durch das sich auf das Herzratenschlagvolumens ergebende Herzzeitvolumens adäquat abgedeckt wird, so übernimmt der Temperatursensor die "Führung", indem die Herzrate jetzt so beeinflußt wird, daß das Herzzeitvolumen der Richtung angepaßt wird.

Das entspricht einem Umschalten der Ausgänge der Kennfelder nach Art einer ODER-Verknüpfung. Die mit dem Schlagvolumen verknüpfte Größe dient jetzt mit dem Produkt der Herzrate zur Ermittlung des tatsächlichen Herzzeitvolumens, wobei die Herzrate soweit angehoben wird, daß das erreichbare Herzzeitvolumen wieder der Belastung entspricht. Das ist bei der Rate $R_2$ der Fall. Die dargestellte Steuerung durch Überlagerung zweier physiologischer Parameter führt dazu, daß der Ratenbereich von $R_0$ bis $R_2$ beim Patienten mit zunehmender Belastung verhältnismäßig schnell durchlaufen wird, um jederzeit ein der Belastung angemessenes adäquates Herzzeitvolumen zur Verfügung zu stellen.

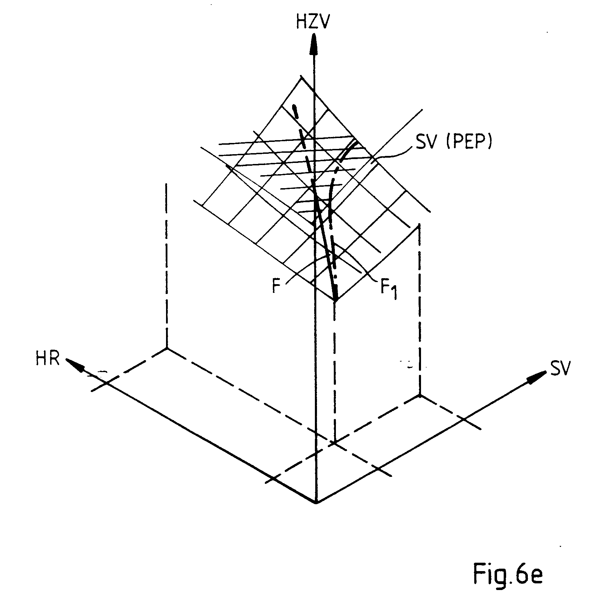

Anhand des in Figur 6e wiedergegebenen Diagramms soll ein Ausführungsbeispiel erläutert werden, bei dem die Umschaltung auf unterschiedliche physiologische Parameter zwecks Steuerung der Herzrate in Abhängigkeit von einem aufgenommenen Parameter, sondern in Abhängigkeit von der Herzrate erfolgt. Bei dem im dargestellten Diagramm wiedergegebenen physiologischen Verlauf verändert sich eine für das Schlagvolumen kennzeichnende Größe von einer bestimmten Herzfrequenz an nicht mehr, obgleich auch bei höheren Frequenzen noch eine Schlagvolumenadaption stattfindet. Dies kann beispielsweise auftreten, wenn zu der FrequenzSteuerung das Signal PEP (Anspannungszeit) herangezogen wird, während LVET (Austreibungszeit) zunächst nicht berücksichtigt wird, sich aber mit zunehmender Belastung noch weiter vergrößert. In diesem Fall würde eine ausschließliche Steuerung in Abhängigkeit von PEP dazu führen, daß die physiologische Regelung bis zu einem bestimmten Herzratenbereich wirksam wird. Oberhalb dieses Wertes erfolgt eine Belastungsanpassung nur noch durch eine physiologische Schlagvolumenadaption, was bei Spitzenbelastungen zu einem unbefriedigenden Adaptionsverhalten führen kann. Auch eine veränderte Programmierung der Kennlinie (F1) in dem zur Verfügung stehenden Arbeitsbereich schafft hier keine Veränderung, da bei einem niedrigeren Anstieg der Herzfrequenz mit Zunahme von PEP lediglich die Schlagvolumenadaption aufgrund von LVEP bereits bei kleineren Raten stärker ausgenutzt wird. In diesem Fall wird (wie beim zuvor dargestellten Diagramm beschrieben) darauf zurückgegriffen werden, daß mittels eines zusätzlichen leistungsbezogenen im Körper des Patienten ermittelten physiologischen Parameters die Herzrate zusätzlich bei Belastung dann heraufgesetzt wird, wenn das Produkt aus Schlagvolumen und Herzrate nicht mehr der körperlichen Belastung adäquat ist. Dazu muß aber dann eine Meßgröße aufgenommen werden, welche mit dem aktuellen Schlagvolumen exakt korreliert ist, während die hier aufgenommene Kenngröße PEP nur für einen Anteil der Schlagvolumenadaption eine Aussage liefert. In diesem Fall wird ein Kennfeld verwendet, welches in Abhängigkeit von der aktuellen Herzrate eine Umschaltung auf die Steuerung mittels eines anderen Parameters besorgt. Die Signale PEP und LVEP bilden dabei mittels Druckfeder Schaallaufnehmer in der kann aufgenommene Signale, die für das Schlagvolumen repräsentativ sind.

Oberhalb einer vorgegebenen Herzrate HR wird damit die Herzfrequenz von der Bluttemperatur gesteuert, so daß eine zusätzliche Herzfrequenzerhöhung bei ansteigender Belastung möglich ist, welche eine weitere Herzfrequenzerhöhung zuläßt. In diesem Fall wird durch ein geeignet programmiertes Kennfeld, welches als Daten eine Verknüpfungsanweisung für unterschiedliche Kennfelder programmiert enthält und dafür sorgt, daß oberhalb eines vorgegebenen Ratenpegels die Bluttemperatur bei einem Wert, der einer starken körperlichen Belastung entspricht, die Bluttemperatur zur weiteren Ratenerhöhung beiträgt, so daß das Herzzeitvolumen der tatsächlichen Belastung angepaßt wird und die zusätzliche Adaptionsfähigkeit des Schlagvolumens (welche bei der Steuerung durch PEP nicht erfaßt zu werden braucht) auch bei höheren Frequenzen eine zusätzliche Reserve bildet. Dabei wird die Bluttemperatur beispielsweise als Differenzwert eine zusätzliche programmierbare relative Änderung der Herzfrequenz erzeugen, so daß auf den Absolutwerten innewohnenden Meßabweichungen keine Rücksicht genommen zu werden braucht. (Eine zusätzliche Messung des absoluten Schlagvolumens ist beispielsweise durch elektroplethysmografische Messung möglich.)

Eine derartige Steuerung hat den Vorteil, daß kurzfristige Anpassungen des Schlagvolumens nicht zu übermäßigen Frequenzsprüngen führen, trotzdem aber an einer langandauernden starken körperlichen Belastung (die Bluttemperatur steigt verzögert) eine zusätzliche Anpassung der Herzleistungsfähigkeit an die Belastung möglich ist.

Bei einer Regelung mit dem Herzzeitvolumen als Bezugsgröße, wobei das aktuelle Schlagvolumen zusammen mit einer Belastungsgröße als Adresse für die Ratendaten im Kennfeld benutzt wird, erfolgt ebenfalls eine entsprechende Ratenerhöhung, wobei LVET oder eine sonstige schlagvolumenabhängige Meßgröße (vorzugsweise Impedanzkardiografie) benutzt werden sollte, um die Schlagvolumenadaption auch im Bereich höherer Belastung zu erfassen. Es ist ersichtlich, daß in Abhängigkeit von Signalpegeln in der dargestellten Weise auch andere Signalverknüpfungen (Steuerung/Regelung) veränderbar sind.

Die vorstehende Beschreibung zeigt, daß die meßtechnischen Verknüpfungen durch Programmierung des Systems zwar einerseits dessen technische Funktion exakt bestimmen und zu einem selbsttätigen Steuer- oder Regelsystem machen - andererseits kann aber der behandelnde Arzt durch Kontrolle der Speicherzuweisungen die Funktionsweise des Systems jederzeit kontrollieren und nach Bedarf eingreifen. Die Erfindung beschränkt sich in ihrer Ausführung nicht auf das vorstehend angegebene Beispiel. Vielmehr sind eine Vielzahl von Varianten denkbar, welche von der dargestellten Lösung auch bei grundsätzlich anders gearteten Ausführungen Gebrauch machen. Insbesondere beschränkt sich die Ausführung nicht auf die Realisierung mit diskreten logischen Baugruppen, sondern läßt sich vorteilhaft auch mit programmierter Logik - insbesondere unter Verwendung eines Mikroprozessors - realisieren.

## Patentansprüche

1. Herzschrittmacher mit Schaltungsmitteln zur Beeinflussung mindestens eines Stimulationsparameters, insbesondere der Stimulationsrate, in Abhängigkeit von einem im Patientenkörper durch mindestens einen ersten Meßwertaufnehmer aufgenommenen Signal, das abhängig von der Körperbelastung ist und das eine Eingangsgröße zur Beeinflussung des Stimulationsparameters bildet,

   **dadurch gekennzeichnet,**

   daß Eichmittel für das im Patientenkörper durch den mindestens einen ersten Meßwertaufnehmer aufgenommene Signal in der Weise vorgesehen sind,

   daß das Ausgangssignal eines zweiten Meßwertnehmers für ein weiteres insbesondere außerhalb des Körpers aufgenommenes Signal für einen vorbestimmten Zeitraum gleichzeitig verarbeitet wird,

   daß Kompensationsmittel während des vorbestimmten Zeitraums in einer dem ersten Meßwertaufnehmer nachgeschalteten Baugruppe so eingefügt werden, daß die erhaltenen Signale vom ersten Meßwertaufnehmer einschließlich der Kompensationsmittel und dem zweiten Meßwertaufnehmer im wesentlichen gleich sind und

   daß die Kompensationsmittel für den nachfolgenden Betriebszeitraum im wesentlichen unverändert bleiben.

2. Herzschrittmacher nach einem der vorangehenden Ansprüche, **dadurch gekennzeichnet,** daß die Schaltungsmittel zur Beeinflussung des Stimulationsparameters durch die in den Speicherplätzen eines Speichers enthaltenen Daten steuerbar sind, wobei die Adressierung der Speicherplätze in Abhängigkeit von einem von der Eingangsgröße abgeleiteten Adressensignal und mindestens einem weiteren Adressensignal erfolgt, das aus einem weiteren mit der körperlichen Belastung korrelierten im Patientenkörper aufgenommenen bzw. einem davon abgeleitetes Signal oder einem unabhängiges Signal gebildet wird, und die in dem Speicher enthaltenen Daten als Ausgangssignal ein Kenndatenfeld zur mindestens mittelbaren Beeinflussung des Schrittmacherparameters bilden, das als Dateneinheit geschlossen adressier-, mit weiteren entsprechenden Kenndatenfeldern verknüpf- und/oder bezüglich seines Dateninhalts graphisch darstellbar ist.

3. Herzschrittmacher nach den Ansprüchen 1, **dadurch gekennzeichnet,** daß die Kompensation darin bestehen, daß die das Ausgangssignal des ersten Meßwertaufnehmers bei veränderlichen Belastungszuständen des Patienten in die gleichzeitig vom Ausgangssignal des zweiten Meßwertaufnehmers adressierten Speicherplätze einbeschrieben werden.

4. Herzschrittmacher nach Anspruch 2, **dadurch gekennzeichnet,** daß die Speicherung unter Zuordnung zu einem weiteren gleichzeitig erscheinenden Signalwert eines anderen Meßwertaufnehmers und/oder der Stimulationsrate erfolgt.

5. Herzschrittmacher nach einem der vorangehenden Ansprüche, **dadurch gekennzeichnet,** daß die Eichung mittels eines während des Zeitraums mit Kommunikationsmitteln verbundenen entsprechenden externen Meßwertaufnehmers erfolgt oder periodisch wiederholt zu durch interne Zeitgebermittel festgelegten Zeitpunkten erfolgt.

6. Herzschrittmacher nach Anspruch 6, **dadurch gekennzeichnet,** daß es sich bei dem externen Meßwertaufnehmer um einen solchen für die körperliche Belastung (Ergometer), das Herzzeitvolumen, den Herzschall-, oder Drucksignale zur Ermittlung des Schlagvolumens über systolische Intervalle und/oder die Atemfrequenz handelt.

7. Herzschrittmacher nach einem der vorangehenden Ansprüche, **dadurch gekennzeichnet,** daß durch die genannte "Eichung" eine Arbeitskennlinie als Steuerkennlinie für den späteren Betrieb festgelegt wird.

8. Herzschrittmacher nach einem der vorangehenden Ansprüche, **dadurch gekennzeichnet,** daß die im Eichzeitraum gefundene Kompensation nur dann in den nachfolgenden Betriebszustand übernommen wird, wenn während des Eichzeitraums keine Störung aufgenommen wird.

9. Herzschrittmacher nach einem der vorangehenden Ansprüche, **dadurch gekennzeichnet,** daß ein Schalt- oder Verknüpfungselement zur Auswahl unterschiedlicher Verarbeitungswege und/oder Meßwertaufnehmer für die von der körperlichen Belastung abhängige Meßgröße vorgesehen ist, wobei die Ansteuerung des Schalt- oder Verknüpfungselements in Abhängigkeit von externen Kommunikationsmitteln, aktuellen Signalwerten, gespeicherten, und insbesondere gemittelten, zuvor erhaltenen Signalwerten und/oder zeitabhängig erfolgt.

10. Herzschrittmacher nach einem der vorangehenden Ansprüche, **dadurch gekennzeichnet,** daß in dem Übertragungsweg für mit der körperlichen Belastung korrelierten Signalen vor oder nach einem nichtlinearen Übertragungsglied ein durch eine oder mehrere Zeitkonstanten und/oder weitere Koeffizienten, entsprechend der Nachbildung eines linearen physikalischen Systems, beschrieben durch eine lineare Differentialgleichung, eine Zeitverzögerung oder einen vorgebbaren zeitabhängigen Verlauf als Bewertungsfaktor oder Absolutwertbegrenzung charakterisiertes lineares zeitabhängiges Übertragungsglied vorgesehen ist.

## Claims

1. Heart pacemaker including circuit means for affecting at least one stimulation parameter, in particular the stimulation rate, in dependence on a signal picked up in the patient's body by at least a first transducer, which is dependent on the body loading and which forms an input magnitude for the control of the stimulation parameter,
characterised in that,

calibration means for the signal picked up in the patient's body by the at least one first transducer are provided in such a manner that,

the output signal of a second transducer for a signal received in particular from outside the body is simultaneously processed for a predetermined period,

that compensation means are inserted in a component group located downstream of the first transducer during the predetermined period such that the signals received from the first transducer including the compensation means and from the second transducer are substantially equal, and

that the compensation means remain substantially unaltered for the succeeding operational period.

2. Heart pacemaker in accordance with any of the preceding claims, characterised in that, the circuit means for affecting the stimulation parameter are controllable by the data contained in the memory positions of a memory, wherein addressing of the memory positions takes place in dependence on an address signal derived from the input magnitude and at least one further address signal which is formed from a further signal picked up in the patient's body and correlated with the body loading, or a signal derived therefrom, or an independent signal, and the data contained in the memory form, as an output signal, a characteristic data field for at least indirectly affecting the heart pacemaker parameter, which is capable of being addressed in a self-contained manner as a data unit, of being combined with further corresponding characteristic data fields, and/or, in so far as its data content is concerned, of being graphically displayed.

3. Heart pacemaker in accordance with the claims 1, characterised in that, the compensation consist in that, [the] the output signal of the first transducer for varying load conditions of the patient are inscribed in the memory positions addressed simultaneously by the output signal of the second transducer.

4. Heart pacemaker in accordance with claim 2, characterised in that, the storage occurs in association with a further simultaneously appearing signal value of another transducer and/or the stimulation rate.

5. Heart pacemaker in accordance with any of the preceding claims, characterised in that, the calibration takes place by means of a corresponding external transducer connected during the period by communications means or periodically repeated at time points determined by internal clock pulse generator means.

6. Heart pacemaker in accordance with claim 6, characterised in that, the external transducer is one as used for the body loading (ergometer), the heart blood volume rate, the cardiac noise, or pressure signals for the determination of the heart beat volume in the systolic interval and/or the breathing rate.

7. Heart pacemaker in accordance with any of the preceding claims, characterised in that, a working characteristic curve serving as a control characteristic curve for the subsequent operation is established by the said "calibration".

8. Heart pacemaker in accordance with any of the preceding claims, characterised in that, the compensation found during the calibration period is only then adopted in the subsequent operational state if no disturbance is recorded during the calibration period.

9. Heart pacemaker in accordance with any of the preceding claims, characterised in that, a switching or combining element is provided for the selection of different processing paths and/or transducers for the measured quantities dependent on the body loading whereby the control of the switching or combining element occurs in dependence on external communication means, current signal values, stored and especially determined previously obtained signal values and/or is time dependent.

10. Heart pacemaker in accordance with any of the preceding claims, characterised in that, in the transmission path for the signals correlated to the body loading, there is provided, before or after a non-linear transmission member, a linear time dependent transmission member which is characterised by one or more time constants and/or other co-efficients corresponding to the simulation of a linear physical system described by a linear differential equation, a time delay or a predetermined time dependent waveform as evaluation factor or absolute limiting value.

**Revendications**

1. Stimulateur cardiaque à circuits permettant d'influer sur au moins un paramètre de stimulation, notamment la fréquence de stimulation, en fonction d'un signal enregistré dans l'organisme du malade par au moins un premier transducteur, signal qui est dépendant de l'effort corporel et forme une grandeur d'entrée pour influer sur le paramètre de stimulation, caractérisé en ce que

sont prévus des étalons pour le signal enregistré dans l'organisme du malade par au moins le premier transducteur, de telle façon

que le signal de sortie d'un second transducteur destiné à un autre signal enregistré notamment extracorporellement est traité simultanément pendant une période prédéterminée,

que des moyens de compensation sont insérés pendant la période prédéterminée dans un module situé en aval du premier transducteur de telle façon que les signaux obtenus en provenance du premier transducteur, y compris des moyens de compensation, ainsi que du second transducteur sont sensiblement identiques et

que les moyens de compensation demeurent sensiblement inchangés pendant la période de fonctionnement suivante.

2. Stimulateur cardiaque selon la revendication précédente, caractérisé en ce que les circuits permettant d'influer sur le paramètre de stimulation peuvent être commandés par les données contenues dans les emplacements de mémoire d'une mémoire, l'adressage des emplacements de mémoire se faisant en fonction d'un signal d'adresse dérivé de la grandeur d'entrée et d'au moins un autre signal d'adresse formé par un autre signal enregistré dans l'organisme du malade et corrélé avec l'effort corporel ou un signal dérivé de ce dernier ou un signal indépendant et les données contenues dans la mémoire sous forme de signal de sortie formant une zone de données caractéristiques pour influer au moins indirectement sur le paramètre du stimulateur cardiaque, laquelle zone sous forme d'unité de données est adressable en bloc, est enchaînable à d'autres zones de données caractéristiques correspondantes et/ou est représentable graphiquement en ce qui concerne le contenu de ses données.

3. Stimulateur cardiaque selon la revendication 1, caractérisé en ce que la compensation consiste en ce que le signal de sortie du premier transducteur est écrit, en cas de variation des états d'effort corporel du malade, dans les emplacements de mémoire adressés simultanément par le signal de sortie du second transducteur.

4. Stimulateur cardiaque selon la revendication 2, caractérisé en ce que la mémorisation s'effectue avec affectation à une autre grandeur de signal d'un autre transducteur, apparaissant simultanément, et/ou à la fréquence de stimulation.

5. Stimulateur cardiaque selon l'une des revendications précédentes, caractérisé en ce que l'étalonnage s'effectue au moyen d'un transducteur externe correspondant relié pendant cette période à des moyens de communication ou de façon périodiquement répétée à des délais définis par des minuteries internes.

6. Stimulateur cardiaque selon la revendication 5, caractérisé en ce que le transducteur externe est un transducteur destiné à l'effort corporel (ergomètre), au débit cardiaque, à l'écho cardiaque, ou à des signaux de pression permettant de déterminer le volume systolique par l'intermédiaire des intervalles systoliques et/ou de la fréquence cardiaque.

7. Stimulateur cardiaque selon l'une des revendications précédentes, caractérisé en ce que ledit "étalonnage" permet de définir une caractéristique de fonctionnement en guise de caractéristique de commande pour le fonctionnement ultérieur.

8. Stimulateur cardiaque selon l'une des revendications précédentes, caractérisé en ce que la compensation trouvée durant la période d'étalonnage n'est adoptée dans l'état de fonctionnement suivant que lorsqu'aucune perturbation n'a été enregistrée durant la période d'étalonnage.

9. Stimulateur cardiaque selon l'une des revendications précédentes; caractérisé en ce qu'est prévu un élément de circuit ou élément logique pour la sélection de voies de traitement différentes et/ou des transdueteurs pour la grandeur mesurée dépendant de l'effort corporel, la commande de l'élément de circuit ou élément logique s'effectuant en fonction de moyens de communications externes, de grandeurs de signaux réelles, de grandeurs de signaux mémorisées, obtenues précédemment, dont on a notamment fait la moyenne, et/ou enfonction du temps.

10. Stimulateur cardiaque selon une des revendications précédentes, caractérisé en ce qu'est prévu dans la voie de transmission pour signaux corrélés avec l'effort corporel, avant ou après un élément de transfert non linéaire, un élément de transfert dépendant du temps, linéaire, caractérisé par une ou plusieurs constantes de temps et/ou d'autres coefficients, selon la copie d'un système physique linéaire, décrit par une équation différentielle linéaire, un retard de temps, ou une évolution prédéterminable en fonction du temps en guise de facteur d'évaluation ou de limitation en valeur absolue.

Fig. 1

Fig. 2

Fig. 3

Fig. 4

Fig.4a

28

Fig.5

Festwert

531

Fig.5a

Fig.6a

Fig.6b

Fig.6c

Fig.6d

Fig.6e